# EUROPEAN PATENT APPLICATION

(11) **EP 4 310 098 A1**
(43) Date of publication of application: **24.01.2024**
(21) Application number: 22770348.5
(22) Date of filing: 08.03.2022
(51) Int. Cl.: C07K 16/00, C12N 15/13, A61K 39/395, A61P 35/00

(54) **SEMENOGELIN NEUTRALIZING ANTIBODY AND EPITOPE AND APPLICATION THEREOF**

(30) Priority: 16.03.2021 CN 202110280166
(71) Applicant: Shanghai Biotroy Biotechnique Co., Ltd., Shanghai 200135 (CN)
(72) Inventor: XU, Jie, Shanghai 200135 (CN); SUN, Yufan, Shanghai 200135 (CN); ZHONG, Jiameng, Shanghai 200135 (CN); ZHAO, Xuehua, Shanghai 200135 (CN)
(74) Representative: Engelhard, Markus
(86) International application number: PCT/CN2022/079756
(87) International publication number: WO 2022/193993

(57) **Abstract**

An antibody that can specifically bind to semenogelin-1 (SEMG1) and semenogelin-2 (SEMG2) and regulate the binding of said proteins with a receptor (LILRB2, LILRB4, CD27, or TIGIT) thereof. It is proven that SEMG1 and SEMG2 have a regulating effect on immune cells, and it is proven that the antibody has a neutralizing effect. A specific linear epitope located on SEMG1 and SEMG2 proteins is provided, and it is proven that an antibody recognizing said epitope has a stronger neutralizing function, can more effectively remove an immunosuppressive effect mediated by SEMG1 and SEMG2, and promotes immune cells to inhibit and kill tumor cells. Further provided are preparation and identification methods for the antibody as well as a biomarker for the administration of such antibody. The present invention has good application prospects in the aspect of preparation of an anti-tumor drug.

## Description

### Cross-Reference to Related Applications

This application claims the priority of the Chinese patent application with the application number 202110280166.3, titled "Semenogelin Neutralizing Antibody and epitope and application thereof' and filed with the Chinese Patent Office on March 16, 2021, the entire contents thereof are hereby incorporated by reference in its entirety in this application.

### Technical Field of the Invention

The invention relates to the field of biomedicine, in particular to a method for inhibiting tumors based on semenogelin and applications thereof.

### Background of the Invention

SEMG1 and SEMG2 belong to semenogelins, which are the most abundant proteins in human semen. SEMG1 and SEMG2 are proteins of 50kda and 63kda, respectively, with 78% similarity at the gene level. They are all secreted into the seminal fluid in the seminal vesicles, and then rapidly degraded into small peptides by prostate-specific antigen (PSA, also known as kallikrein peptidase). SEMG proteins and their proteolytic products have many important functions. They regulate sperm motility and fertility and provide sperm with antimicrobial defense. At the same time, SEMG1 and SEMG2 belong to cancer-testis antigen (CTA), which is a group of proteins frequently expressed in various tumors but usually limited to germ cells. Recent studies have shown that SEMGs have the effect of anti-tumor cell proliferation. At the same time, our recent studies have found that SEMG2 is capable of binding to CD27 molecules, and antibody molecules against SEMG2 are active in PBMC killing tumor cells *in vitro* and mouse tumor models.

CD27 molecule belongs to the tumor necrosis factor receptor (TNFR) superfamily member, is a type I membrane protein with a molecular weight of about 55kDa, and exists as a dimer in which two monomers are linked by disulfide bonds. CD27 is mainly expressed on lymphocytes, and activation of the CD27 signaling pathway can increase the infiltration of regulatory T cells (Treg) in solid tumors and reduce antitumor immunity (Claus C, Riether C, Schürch C, Matter MS, Hilmenyuk T, Ochsenbein AF. Cancer Res. 2012 Jul 15;72(14):3664-76). Consistent with this, the study also found that Treg cells in skin tissue could not perform normal immune regulation functions after losing CD27 expression (Remedies KA, Zirak B, Sandoval PM, Lowe MM, Boda D, Henley E, etc., Sci Immunol.2018 Dec 21; 3(30). pii: eaau2042). Furthermore, activation of CD27 increases the number of Tregs and reduces atherosclerosis in hyperlipidemic mice (Winkels H, Meiler S, Lievens D, Engel D, Spitz C, Bürger C, et al., Eur Heart J. 2017; 38(48):3590-3599). The recent studies mentioned above consistently show that CD27 plays an important role in the functional activation of specific Treg cells, including tumor-infiltrating Treg cells, thus avoiding activation of CD27 expressed by tumor-infiltrating Treg cells is a potential cancer treatment strategy. Binding to the ligand can activate the downstream signal transduction of CD27, and the currently known ligand molecule of CD27 is CD70. Currently, blocking the binding of CD70 and CD27 is a tumor immunotherapy strategy which is being studied. In the published information, there is no report about SEMG1 or SEMG2 being the ligand of CD27.

LILRB2 and LILRB4 belong to the family of leukocyte Ig-like receptors (LILRs) and play a very important role in the function of the immune system. LILRB2, a class of immune system inhibitory receptors, is mainly expressed in myeloid cells and B cells, and is involved in the negative regulation of their functions. The endogenous ligand of LILRB2 is an MHC molecule, binding to which produces an inhibitory signal as a mechanism to buffer the immune response and maintain immune tolerance. Genetic studies have shown that tumor-associated macrophages (TAMs) in various tumor microenvironments highly express LILRB2, and inhibiting LILRB2 reduces the invasion of Treg and MDSC in tumor tissues. LILRB4 is mainly expressed on APCs as an immune tolerance receptor. LILRB4-expressing APCs play a key role in controlling inflammation by inhibiting the expression of co-stimulatory molecules. LILRB4 is associated in various diseases such as Kawasaki disease and systemic lupus erythematosus (SLE), inflammatory diseases, as well as some neurological diseases such as multiple sclerosis. LILRB4 is also an important marker of monocytic leukemia, supporting tumor cell infiltration into tissues and inhibiting T cell activity in acute myeloid leukemia (AML) cells. In the published information, there is no report that SEMG1 or SEMG2 is the ligand of LILRB2 or LILRB4.

TIGIT (also known as WUCAM, Vstm3, VSIG9) is a receptor of the Ig superfamily consisting of an extracellular immunoglobulin (Ig) variable domain, a type 1 transmembrane domain and an intracellular domain. The intracellular domain has two inhibitory motifs that are conservative in mice and humans: the immunoreceptor tyrosine-based inhibitory motif (ITIM) and the Ig tail tyrosine-like (ITT) motif. TIGIT is expressed by activated CD8+ T and CD4+ T cells, natural killer (NK) cells, regulatory T cells (Tregs), and follicular helper T cells. TIGIT functions as a negative regulator of T cells, acting as a brake. TIGIT potentially suppresses innate and adaptive immunity through multiple mechanisms. TIGIT acts on Tregs to enhance immunosuppressive function and stability. In the published information, there is no report about SEMG1 or SEMG2 being the ligand of TIGIT.

We found that SEMG1 and SEMG2 proteins are expressed in tumor cells, and both can act as ligands to bind LILRB2, LILRB4, CD27 and TIGIT receptors, thereby inhibiting the killing of tumor cells by T cells and inducing the expansion of Tregs and MDSCs. These results suggest that only two members of the semenogelin family, SEMG1 and SEMG2, have the function of promoting tumor immune escape. Based on the above-mentioned original scientific discovery, the present invention innovatively developed a dual-targeting antibody that is capable of binding SEMG1 and SEMG2, which can bind the above-mentioned target protein with high affinity and neutralize its function, and inhibits its binding with one or more ligands, so as to achieve the effect of releasing the immune escape function of tumor cells, and to promote the killing of tumor cells by immune cells *in vivo* and *in vitro.* The antibody described in the present invention can be used as an active ingredient to develop a medicament for treating tumors. At the same time, the present invention also discloses technical solutions closely related to the disclosed antibodies, such as the linear epitope corresponding to the antibody with strong neutralizing function, the screening method of the antibody, and the biomarker for the administration of the antibody.

### Summary of the Invention

While the invention may be embodied in many different forms, disclosed herein are specific illustrative embodiments thereof that demonstrate the principles of the invention. It should be emphasized that the invention is not limited to the particular embodiments illustrated. Furthermore, any section headings used herein are for organizational purposes only and are not to be construed as limiting the described subject matter.

Unless otherwise defined hereinafter, all technical and scientific terms used in the detailed description of the invention have the same meanings as commonly understood by the skilled person in the art. While the following terms are believed to be well understood by the skilled person in the art, the following definitions are set forth to better explain the invention.

The terms "including", "comprising", "having", "containing" or "involving" are inclusive or open-ended, and do not exclude other unrecited elements or method steps. The term "consisting of ..." is considered as a preferred embodiment of the term "comprising". If a group is hereinafter defined as comprising at least a certain number of embodiments, this should also be understood as revealing a group preferably consisted of these embodiments only.

The use of an indefinite or definite article when referring to a noun in the singular form, e.g., "a" or "an", "the", includes a plural form of the noun.

In addition, the terms first, second, third, (a), (b), (c) and the like in the description and claims are used to distinguish similar elements, and are not necessary to describe the order or time sequence. It is to be understood that the terms so used are interchangeable under appropriate circumstances, and that the embodiments described herein can be carried out in other sequences than described or illustrated herein.

The term "and/or" is considered a specific disclosure that each of the two specified features or components is with or without the other. Therefore, the term "and/or" as used herein in phrases such as "A and/or B" is intended to include A and B; A or B; A (alone); and B (alone). Likewise, the term "and/or" as used in phrases such as "A, B, and/or C" is intended to encompass each of the following: A, B, and C; A, B, or C; A, or C; A or B; B or C; A and C; A and B; B and C; A (alone); B (alone); and C (alone).

The terms "for example" and "i.e." are used by way of example only, not intended to be limiting, and should not be construed as referring to only those items explicitly recited in the description.

The terms "or more", "at least", "more than" etc., for example "at least one" should be understood to include but not limited to at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or 200, 300, 400, 500, 600, 700, 800, 900, 1000, 2000, 3000, 4000, 5000 or more than the stated value. Any higher numbers or fractions in between are also included.

Conversely, the term "no more than" includes every value that is less than the stated value. For example, "no more than 100 nucleotides" includes 100, 99, 98, 97, 96, 95, 94, 93, 92, 91, 90, 89, 88, 87, 86, 85, 84, 83, 82, 81, 80, 79, 78, 77, 76, 75, 74, 73, 72, 71, 70, 69, 68, 67, 66, 65, 64, 63, 62, 61, 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1 and 0 nucleotides. Any lower numbers or fractions in between are also included.

The terms "a plurality", "at least two", "two or more", "at least a second" etc., should be understood to include but are not limited to, at least 2, 3, 4, 5, 6, 7, 8, 9, 10, 11, 12, 13, 14, 15, 16, 17, 18, 19 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or 200, 300, 400, 500, 600, 700, 800, 900, 1000, 2000, 3000, 4000, 5000 or more. Any higher numbers or fractions in between are also included.

The terms "about" and "approximately" represent the range of accuracy that can be understood by the skilled person in the art which still guarantee the technical effect of the mentioned feature. The term generally means a deviation of ±10%, preferably ±5%, of the indicated value.

As stated herein, unless otherwise indicated, any concentration range, percentage range, ratio range, or integer range should be understood to include the value of any integer within the stated range and, where appropriate, fractions thereof (e.g., tenths and hundredths of an integer).

As used herein, the term "antibody" (Ab) includes, but is not limited to, a glycoprotein immunoglobulin that specifically binds to an antigen. Typically, an antibody may comprise at least two heavy (H) chains and two light (L) chains linked by disulfide bonds, or an antigen-binding molecule thereof. Each H chain comprises a heavy chain variable region (abbreviated herein as VH) and a heavy chain constant region. The heavy chain constant region comprises three constant domains, CH1, CH2 and CH3. Each light chain comprises a light chain variable region (abbreviated herein as VL) and a light chain constant region. The light chain constant region comprises one constant domain, CL. The VH and VL regions can be further subdivided into hypervariable regions called complementarity determining regions (CDRs), and interspersed with more conserved regions called framework regions (FRs). Each VH and VL comprises three CDRs and four FRs, arranged from amino-terminus to carboxy-terminus in the following order: FR1, CDR1, FR2, CDR2, FR3, CDR3, and FR4. The variable regions of the heavy and light chains comprise binding domains that interact with the antigen. The constant regions of Ab can mediate the binding of the immunoglobulin to host tissues or factors, including various cells of the immune system (e.g., effector cells) and the first component (C1q) of the classical complement system.

The light and heavy chain variable regions each comprise a "framework" region interspersed with three hypervariable regions (also called "complementarity determining regions" or "CDRs"). A "complementarity determining region" or "CDR region" or "CDR" or "hypervariable region" (used interchangeably herein with hypervariable region "HVR"), is a region that is hypervariable in sequence and forms structurally defined loops ("hypervariable loops") and/or contains antigen contact residues ("antigen contact points") in the antibody variable domain. The CDRs are primarily responsible for binding to antigenic epitopes. The CDRs of the heavy and light chains are commonly referred to as CDR1, CDR2 and CDR3, numbered sequentially starting from the N-terminus. The CDRs located within the variable domain of an antibody heavy chain are referred to as HCDR1, HCDR2, and HCDR3, while the CDRs located within the variable domain of an antibody light chain are referred to as LCDR1, LCDR2, and LCDR3. In the amino acid sequence of a given light chain variable region or heavy chain variable region, the precise amino acid sequence boundaries of each CDR can be determined using any one or combination of a number of well-known antibody CDR assignment systems, including for example, Chothia based on the three-dimensional structure of antibodies and the topology of the CDR loops (Chothia et al. (1989) Nature 342:877-883, Al-Lazikani et al., "Standard conformations for the canonical structures of immunoglobulins", Journal of Molecular Biology, 273, 927-948 (1997)), Kabat based on antibody sequence variability (Kabat et al. Human, Sequences of Proteins of Immunological Interest, 4th Edition, U.S. Department of Health and Human Services, National Institutes of Health (1987)), AbM (University of Bath), Contact (University College London), International ImMunoGeneTics database (IMGT), and the North CDR definition based on affinity propagation clustering using a large number of crystal structures.

However, it should be noted that the boundaries of the CDRs of the variable region of the same antibody obtained based on different assignment systems may differ. That is, the CDR sequences of the variable region of the same antibody defined under different assignment systems are different. For example, the residue ranges defined by different assignment systems for CDR regions numbered by Kabat and Chothia are shown in Table A below.

**Table A. CDR residues ranges defined by different assignment systems**

| Loop | Kabat CDR | AbM | Chothia | Contact | IMGT |
|---|---|---|---|---|---|
| L1 | L24-L34 | L24-L34 | L24-L34 | L30-L36 | L27 L32 |
| L2 | L50-L56 | L50-L56 | L50-L56 | L46-L55 | L50-L52 |
| L3 | L89-L97 | L89-L97 | L89-L97 | L89-L96 | L89-L96 |
| H1 | H31-H35b Kabat Numbering | H26-H35b | H26-H32...34 | H30-H35b | H26-H35b |
| H1 | H31-H35 Chothia Numbering | H26-H35 | H26-H32 | H30-H35 | H26-H35 |
| H2 | H50-H65 | H50-H58 | H52-H56 | H47-H58 | H51-H57 |
| H3 | H95-H102 | H95-H102 | H95-H102 | H93-H101 | H93-H102 |

Therefore, relating to defining antibodies with particular CDR sequences as defined in the present invention, the scope of the antibody also covers antibodies whose variable region sequences comprise the particular CDR sequences, but due to the application of a different scheme (e.g. different assignment system rules or combinations), the claimed CDR boundaries are different from the specific CDR boundaries defined in the present invention.

The CDRs of the antibodies of the invention can be manually assessed to determine the boundaries according to any protocol in the art or a combination thereof. Unless otherwise stated, in the present invention, the term "CDR" or "CDR sequence" covers the CDR sequences determined in any of the above manners.

Antibodies can include, for example, monoclonal antibodies, recombinant antibodies, monospecific antibodies, multispecific antibodies (including bispecific antibodies), human antibodies, engineered antibodies, humanized antibodies, chimeric antibodies, immunoglobulins, synthetic antibodies, tetrameric antibody comprising two heavy chain and two light chain molecules, antibody light chain monomer, antibody heavy chain monomer, antibody light chain dimer, antibody heavy chain dimer, antibody light chain-antibody heavy chain pair, intrabody, antibody fusion (sometimes referred to herein as "antibody conjugate"), heteroconjugated antibody, single domain antibody, monovalent antibody, single chain antibody or single chain Fv (scFv), camelized antibody, affibody, Fab fragment, F(ab')₂ fragment, disulfide-linked Fv (sdFv), anti-idiotypic (anti-Id) antibody (including, for example, anti-anti-Id antibodies), minibody, domain antibody, synthetic antibody (sometimes referred to herein as "antibody mimetic"), and antigen-binding fragment of any of the above.

The term "humanized antibody" is intended to mean an antibody obtained by grafting a CDR sequence derived from the germline of another mammalian species, such as a mouse, into human framework sequences. Additional framework region modifications can be made in the human framework sequences.

As used herein, "antigen-binding molecule", "antigen-binding fragment" or "antibody fragment" refers to any molecule comprising an antigen-binding fragment (e.g., CDR) of an antibody from which the molecule is derived. Antigen binding molecules may include complementarity determining regions (CDRs). Examples of antibody fragment include, but are not limited to, Fab, Fab', F(ab')₂ and Fv fragments, dAb, linear antibody, scFv antibody and multispecific antibody formed from an antigen binding molecule. In some embodiments, the antigen-binding molecule binds to SEMG1 protein. In some embodiments, the antigen-binding molecule has neutralizing activity and can inhibit the binding of SEMG1 or SEMG2 to the receptor CD27, LILRB2, LILRB4 or TIGIT.

As used herein, the term "antigen" refers to any molecule that elicits an immune response or is capable of being bound by an antibody or antigen binding molecule. The immune response may involve antibody production or activation of specific immunocompetent cells, or both. The skilled person in the art will readily understand that any macromolecule, including almost any protein or peptide, can function as an antigen. Antigens may be expressed endogenously, i.e., from genomic DNA, or may be recombinantly expressed. An antigen may be specific for certain tissues, such as cancer cells, or it may be expressed broadly. In addition, fragments of larger molecules can function as antigens. In some embodiments, the antigen is a SEMG1 or SEMG2 protein antigen.

As used herein, in some embodiments, the antigen binding molecule, scFv, antibody or fragment thereof directly blocks the binding site on the ligand or alters the binding ability of the ligand through indirect means such as structural or energetic changes of the ligand. In some embodiments, the antigen binding molecule, scFv, antibody or fragment thereof prevents the biological function of the protein to which it binds.

As used herein, the terms "peptide", "polypeptide" and "protein" are used interchangeably and refer to a compound comprising amino acid residues covalently linked by peptide bonds. A protein or peptide contains at least two amino acids and there is no limit to the maximum number of amino acids that can be comprised in the sequence of a protein or peptide. Polypeptides include any peptide or protein comprising two or more amino acids linked to each other by peptide bonds. As used herein, the term refers to both of short chains (which are also commonly referred to in the art as e.g. peptides, oligopeptides, and oligomers) and longer chains (which are commonly referred to in the art as proteins, which have many types). "Polypeptide" includes, for example, biologically active fragments, substantially homologous polypeptides, oligopeptides, homodimers, heterodimers, variants of polypeptides, modified polypeptides, derivatives, analogs, fusion proteins, etc. Polypeptides include natural peptides, recombinant peptides, synthetic peptides or combinations thereof.

As used herein, the term "specifically binds" or "specifically binds to" refers to a non-random binding reaction between two molecules, such as between an antibody and an antigen.

As used herein, the ability to "inhibit binding", "block binding" or "compete for the same epitope" refers to the ability of an antibody to inhibit the binding of two molecules to any detectable extent. In some embodiments, an antibody that blocks the binding between two molecules inhibits the binding interaction between the two molecules by at least 50%. In some embodiments, the inhibition may be greater than 60%, greater than 70%, greater than 80%, or greater than 90%.

As used herein, the term "Ka" is intended to denote the association rate of a particular antibody-antigen interaction, while the term "Kd" as used herein is intended to denote the dissociation rate of a particular antibody-antigen interaction. As used herein, the term "K_{D}" or "K_{D} value" is intended to mean the dissociation constant for a particular antibody-antigen interaction, which is obtained from the ratio of Kd to Ka (i.e., Kd/Ka) and expressed as a molar concentration (M). K_{D} values of antibodies can be determined using methods well established in the art.

As used herein, the term "high affinity" antibody refers to antibodies against a target antigen with a K_{D} value of 1×10⁻⁷ M or lower, more preferably 5×10⁻⁸ M or lower, even more preferably 1×10⁻⁸ M or lower, even more preferably 5×10⁻⁹ M or lower, and even more preferably 1×10⁻⁹ M or lower.

As used herein, the term "epitope" refers to the antigen portion to which an immunoglobulin or antibody specifically binds. An "epitope" is also referred to as an "antigenic determinant". Epitope or antigenic determinant usually consists of chemically active surface groups of molecules such as amino acids, carbohydrates or sugar side chains, and usually have a specific three-dimensional structure and specific charge characteristics. For example, an epitope typically comprises at least 3, 4, 5, 6, 7, 8, 9, 10, 11, 12, 13, 14, or 15 consecutive or nonconsecutive amino acids in a unique three-dimensional conformation, which may be a "linear epitope" or "conformational epitope". See e.g., Epitope Mapping Protocols in Methods in Molecular Biology, Vol. 66, G.E. Morris, Ed. (1996). In a linear epitope, all interaction sites between a protein and an interacting molecule (such as an antibody) occur linearly along the primary amino acid sequence of the protein. In a conformational epitope, the interaction sites span across amino acid residues that are separated from each other in the protein. Antibodies can be screened depending on their competition for binding to the same epitope as detectable by conventional techniques known to the skilled person in the art. For example, competition or cross-competition studies can be performed to obtain antibodies that compete or cross-compete with each other for binding to an antigen (e.g., CLDN18.2). A high-throughput method for obtaining antibodies binding to the same epitope, based on their cross-competition, is described in International Patent Application WO 03/048731.

The term "nucleic acid" or "nucleic acid sequence" in the present invention refers to any molecule, preferably a polymeric molecule, comprising ribonucleic acid, deoxyribonucleic acid or analog units thereof. The nucleic acid can be single-stranded or double-stranded. A single-stranded nucleic acid may be the nucleic acid of one strand of denatured double-stranded DNA. Alternatively, a single-stranded nucleic acid may be a single-stranded nucleic acid that is not derived from any double-stranded DNA.

The term "complementary" as used herein refers to the hydrogen bonding base pairing between the nucleotide bases G, A, T, C and U, such that when two given polynucleotides or polynucleotide sequences are annealed to each other, in DNA, A is paired with T, G is paired with C, and in RNA, G is paired with C, A is paired with U.

As used herein, the term "cancer" refers to a large group of various diseases characterized by the uncontrolled growth of abnormal cells in the body. Unregulated cell division and growth lead to the formation of malignant tumors that invade adjacent tissues and can also metastasize to remote parts of the body through the lymphatic system or bloodstream. "Cancer" or "cancerous tissue" may include tumors. For example, bone cancer, pancreatic cancer, skin cancer, head or neck cancer, skin or eye malignant melanoma, uterine cancer, ovarian cancer, rectal cancer, anal region cancer, gastrointestinal cancer, testicular cancer, uterine cancer, fallopian tube cancer, endometrial cancer, cervical cancer, vaginal cancer, vulvar cancer, Hodgkin's disease, non-Hodgkin's lymphoma, esophagus cancer, small intestine cancer, endocrine system cancer, thyroid cancer, parathyroid cancer, adrenal gland cancer, soft tissue sarcoma, urethral cancer, penile cancer, chronic or acute leukemia (including acute myeloid leukemia, chronic myeloid leukemia, acute lymphoblastic leukemia, chronic lymphocytic leukemia), childhood solid tumors, lymphocytic lymphoid tumor, bladder cancer, kidney or ureter cancer, renal pelvis cancer, central nervous system (CNS) neoplasm/tumor, primary CNS lymphoma, tumor angiogenesis, spinal axis tumor, brainstem glioma, pituitary adenoma, Kaposi's sarcoma, epidermoid carcinoma, squamous cell carcinoma, T-cell lymphoma, environmentally induced cancers (including those induced by asbestos), and combinations thereof.

As used herein, the term "cancer associated with SEMG1 or SMEG2" refers to any cancer caused by, aggravated by, or otherwise associated with increased or decreased expression or activity of SEMG1 or SEMG2. In some embodiments, the method disclosed herein can be used for cancers selected from the group consisting of colorectal cancer, lung cancer, breast cancer, melanoma, lymphoma, liver cancer, head and neck cancer, gastric cancer, kidney cancer, bladder cancer, prostate cancer, testicular cancer, endometrial cancer, breast cancer, and ovarian cancer.

As used herein, an "effective dose", "effective amount" or "therapeutically effective dose" is any amount that protects a subject from onset of disease or promotes regression of disease when used alone or in combination with another therapeutic agent. The regression of a disease is evidenced by a decrease in the severity of disease symptoms, an increase in the frequency and duration of asymptomatic periods of disease, or prevention of impairment or disability due to disease affliction. The ability of a therapeutic agent to promote disease regression can be assessed using various methods known to the skilled person in the art, for example, in human subjects during clinical trials, in animal model systems for prediction of efficacy in human, or by determining activity of reagents in *in vitro* assays.

As used herein, an "individual" or "subject" is a mammal. Mammals include primates (e.g., humans and non-human primates such as monkeys) and rodents (e.g., mice and rats). In certain embodiments, the individual or subject is a human. A "subject" may be a "patient", who is a human subject in need of treatment, and may be an individual with or at risk of developing a SEMG1 or SEMG2 related cancer, such as breast cancer.

As used herein, the term *"in vitro* cell" refers to any cell cultured *ex vivo.* In particular, *in vitro* cells may include T cells.

As used herein, the term "pharmaceutically acceptable" means that the carrier, diluent, excipient and/or salt thereof is chemically and/or physically compatible with the other ingredients in the formulation and physiologically compatible with the recipient.

As used herein, the term "pharmaceutically acceptable carrier and/or excipient" refers to a carrier and/or excipient that is pharmacologically and/or physiologically compatible with the subject and the active agent, which is well known in the art (*See*, e.g., Remington's Pharmaceutical Sciences. Edited by Gennaro AR, 19th ed. Pennsylvania: Mack Publishing Company, 1995), and include but are not limited to pH adjusters, surfactants, adjuvants and ionic strength enhancers. For example, pH adjusters include but are not limited to phosphate buffer; surfactants include but are not limited to cationic, anionic or nonionic surfactants such as Tween-80; ionic strength enhancers include but are not limited to sodium chloride.

As used herein, the term "regulation" generally includes the meaning of up-regulation or down-regulation in two opposite directions. In some cases, it can be understood as inhibition or enhancement; in some cases, it can be understood as reduction or improvement; in some cases, it can be understood as reduction or increase, etc., and the specific explanation is not limited and can be understood and interpreted according to the actual application context. For example, in some embodiments, "regulating" tumor cell growth can be understood as inhibiting or enhancing tumor cell growth.

As used herein, the terms "reduce" and "decrease" are used interchangeably and mean any change that is smaller than the original. "Reduce" and "decrease" are relative terms that require a comparison between before and after measurements. "Reduce" and "decrease" include total consumption; likewise, the terms "enhance" and "increase" are opposite interpretations.

"Treatment" or "treating" of a subject means performing any type of intervention or procedure on a subject, or administering an active agent to a subject, in order to reverse, alleviate, ameliorate, inhibit, relieve or prevent symptoms, complications or conditions, or the onset, progression, development, severity, or recurrence of disease-related biochemical indicators. In some embodiments, "treatment" or "treating" includes partial remission. In another embodiment, "treatment" or "treating" includes complete remission.

Various aspects of the disclosure are described in further detail:

### 1. SEMG1 or SEMG2 Proteins and Their Roles in Tumors

Protein SEMG1 or SEMG2 both belong to SEMG proteins.

However, the function of SEMG1 or SEMG2 protein has not been deeply understood, and there is no report on the inhibition of tumors based on SEMG1 or SEMG2. The inventors have confirmed through gene silencing knockout and immunology experiments that by inhibiting the function or activity of SEMG1, the interaction of SEMG1 or SEMG2 with receptors CD27, LILRB2, LILRB4 or TIGIT can be blocked and the killing effect of immune cells on tumor cells can be promoted, so that it can be used in the field of cancer treatment.

In some embodiments, the cancer is a cancer associated with SEMG1 or SEMG2.

As used herein, the term "cancer associated with SEMG1 or SEMG2" refers to any cancer caused by, aggravated by, or otherwise associated with increased or decreased expression or activity of SEMG1 or SEMG2.

In some embodiments, the methods disclosed herein can be used for cancers selected from colorectal cancer, lung cancer, breast cancer, melanoma, lymphoma, liver cancer, head and neck cancer, gastric cancer, kidney cancer, bladder cancer, prostate cancer, testicular cancer, endometrial cancer, breast cancer, and ovarian cancer.

### 2. Method for Tumor Cell Regulation Based on SEMG1 or SEMG2

Based on the above findings of the present invention, a method for regulating tumor cells *in vitro* or *in vivo* is disclosed. The method changes the cell ratio of immunosuppressive cells MDSC or Treg, and regulates the killing effect of PBMC on tumor cells by changing the binding effect of SEMG1 or SEMG2 to any one of the receptors CD27, LILRB2, LILRB4 or TIGIT.

It is understandable in the art that such regulation can regulate the cell activity of tumor cells, or the growth of tumor cells, or the migration of tumor cells, etc., and there is no limitation here. It is reasonable to expect that such regulation can be in various aspects in the field. "Regulation" includes two aspects of enhancement and inhibition. In more embodiments, this regulation is the aspect of inhibition, that is, the inhibition of tumor cell activity, growth or migration, etc., in order to achieve the purpose of disease focus removal or treatment purposes.

In some specific inhibition embodiments, the method may comprise the steps of:
a) For tumor cells expressing SEMG1 or SEMG2, the above-mentioned purpose is achieved by inhibiting the expression, function or activity of SEMG1 or SEMG2. Optionally, before step a), detection of SEMG1 or SEMG2 expression of tumor cells may also be included, so as to better realize the above method.

Further, the method also includes step b):
b) contacting tumor cells with immune cells; it will be understood that step b) can be performed before or after step a), without affecting the implementation of the method.

It is understood in the art that there are many methods for inhibiting the expression, function or activity of a specific protein, and these methods can be carried out at the protein level or gene level, which are well known in the art.

In some embodiments, the above-mentioned inhibition method can be any of the following:
a. Gene editing of the SEMG1 or SEMG2 gene to destroy the integrity of the gene, thereby reducing the expression and activity of the gene, etc.;
   In some preferred embodiments, the gene editing system of the present invention is a CRISPR/Cas9 gene editing system;
   In some more preferred embodiments, in the CRISPR/Cas9 gene editing system, the oligomeric DNA sequence for encoding sgRNA is selected from SEQ ID NOs: 1-8.
b. Interfering the expression of SEMG1 or SEMG2 gene, by way such as RNAi, to reduce the expression or activity of the gene;
c. Inhibition by immune means, such as inhibiting the function or activity of SEMG1 or SEMG2 protein through antibodies.

On the other hand, the present invention can also provide other regulation methods, for example, methods for regulating CD27, LILRB2, LILRB4 or TIGIT activity in cells *in vivo* and *in vitro;* for example, methods for regulating the ratio of immunosuppressive cells MDSC or Treg cells *in vivo* and *in vitro;* another example, methods for regulating the killing effect of PBMC on tumor cells *in vivo* and *in vitro.* These methods all include the step of altering the function or activity of SEMG1 or SEMG2 in tumor cells.

In some embodiments, the above regulation method is a method for inhibiting the activity of CD27, LILRB2, LILRB4 or TIGIT; a method for reducing the ratio of immunosuppressive cells MDSC or Treg cells; a method for enhancing the killing effect of PBMC on tumor cells. These methods respectively include the step of inhibiting the function or activity of SEMG1 or SEMG2 in tumor cells.

### 3. Inhibitor of the Present Invention and Screening or Identifying Method Thereof

Based on the discovery of the present invention that SEMG1 or SEMG2 proteins can bind to receptors CD27, LILRB2, LILRB4 or TIGIT and the like, and the expression of SEMG1 or SEMG2 proteins in tumor cells can affect myeloid-derived suppressor cells (MDSC), regulatory T cells (Treg) and immune cells.

The present invention provides a SEMG1 or SEMG2 inhibitor. The "inhibitor" in the present invention can be a protein-level inhibitor or a gene-level inhibitor, and any gene or protein that can inhibit SEMG1 or SEMG2 gene or protein, including but not limited to the production, transcription, activity, function, and the like of gene, as well as the expression, activity, and functions and the like of protein, all belong to the category of "inhibitors".

Exemplarily, as a protein-level inhibitor, it is a substance that can bind to SEMG1 or SEMG2 protein, and simultaneously inhibit or block the interaction of SEMG1 or SEMG2 with any one of CD27, LILRB2, LILRB4 or TIGIT.

In some embodiments, the protein-level SEMG1 or SEMG2 inhibitor specifically binds to a SEMG1 or SEMG2 protein.

In some embodiments, the protein-level SEMG1 or SEMG2 inhibitor binds to a SEMG1 or SEMG2 protein with specificity and high affinity.

In some embodiments, the protein-level inhibitor is a compound that agonizes or antagonizes the interaction of SEMG1 or SEMG2 with CD27, LILRB2, LILRB4 or TIGIT.

In some embodiments, the protein-level inhibitors include, but are not limited to, small molecule inhibitors, polypeptides, antibodies, or antigen-binding fragments.

As a gene-level inhibitor, it is a substance that can alter the activity or function of SEMG1 gene, thereby inhibit or block the interaction of SEMG1 or SEMG2 with any one of CD27, LILRB2, LILRB4 or TIGIT.

In some embodiments, the gene-level SEMG1 or SEMG2 inhibitor includes, but is not limited to, small molecule substances, DNA-like substances or RNA-like substances that specifically target the SEMG1 gene, such as primers, probes, sgRNA and other substances.

The present invention also provides a method for screening or identifying the above-mentioned inhibitors, which may include the following steps:
First obtain the SEMG1 inhibitory molecule, and then analyze the effect of the candidate molecule on the binding of SEMG1 to the receptor or LILRB2; preferably, analyze whether the candidate molecule has antagonistic activity.

In some embodiments, the method may include the steps of:

First obtain a SEMG1 or SEMG2 inhibitory molecule, and then analyze the effect of the candidate molecule on myeloid-derived suppressor cells (MDSC); preferably, analyze whether the candidate molecule can reduce the content or ratio of suppressor cells (MDSC).

In some embodiments, the method may include the following steps: first obtain a SEMG1 inhibitory molecules, and then analyze the effect of the candidate molecule on regulatory T cells (Treg); preferably, analyze whether the candidate molecule can reduce the content or ratio of regulatory T cells (Treg).

In some embodiments, the method may include the following steps: first obtain a SEMG1 inhibitory molecule, and then analyze the effect of the candidate molecule on immune cells killing tumor cells; preferably, analyze whether the candidate molecule can promote the killing or inhibition effect of immune cells on tumor cells.

In some embodiments, the method may include a combination or all of the above steps.

Through the above methods, it can be expected in the art to screen and/or identify valuable SEMG1 inhibitory molecules.

### 4. Antibody of the Present Invention and Preparation Method Thereof

### Antibodies of the Present Invention

The terms "antibody against SEMG1 or SEMG2", "anti-SEMG1 or SEMG2 antibody", "anti-SEMG1 or SEMG2 protein antibody", "SEMG1 or SEMG2 protein antibody", or "antibody binding to SEMG1 or SEMG2" are used interchangeably herein, and refer to an antibody of the present invention that is capable of binding to SEMG1 or SEMG2 protein with sufficient affinity, whereby the antibody can be used as a diagnostic, preventive and/or therapeutic agent targeting SEMG1 or SEMG2 protein.

Any antibody or antigen-binding fragment thereof having the following properties theoretically belongs to the scope of inventive concept or the claims of the present invention, specifically, the isolated antibody or antigen-binding fragment thereof is:
a) capable of specifically binding to a SEMG1 or SEMG2 protein; and
b) capable of inhibiting or blocking the interaction of SEMG1 or SEMG2 with any one of CD27, LILRB2, LILRB4 or TIGIT.

In some embodiments, the antibody further comprises at least one of the following properties:
the antibody reduces the ratio of immunosuppressive cells MDSC cells;
the antibody reduces the cell ratio of immunosuppressive cells Treg; or
the antibody enhances the killing effect of immune cells (such as PBMC) on tumor cells.

Additionally/alternatively, the antibody may comprise one or more of the other characteristics as mentioned above.

In some specific embodiments, the isolated antibody or antigen-binding fragment thereof of the present invention comprises a heavy chain variable region and a light chain variable region, and the specific sequences may be as follows:
the three CDRs in the amino acid sequence of the heavy chain variable region as shown in SEQ ID NO: 13 and the three CDRs in the amino acid sequence of the light chain variable region as shown in SEQ ID NO: 14; or a variant with no more than two amino acid variations in each CDR region in single or multiple CDRs of the six CDRs.

Wherein the amino acid variation is an addition, deletion or substitution of amino acids, preferably, the amino acid variation is a conservative amino acid substitution.

In some embodiments, the antibody comprises a heavy chain variable region of HCDR1, HCDR2 and HCDR3 sequences as defined according to IMGT; and a light chain variable region of LCDR1, LCDR2 and LCDR3 sequences as defined according to IMGT, the sequences are as follows:
i. the amino acid sequence of the HCDR1 is shown in SEQ ID NO. 15;
ii. the amino acid sequence of the HCDR2 is shown in SEQ ID NO.16;
iii. the amino acid sequence of the HCDR3 is shown in SEQ ID NO.17;
iv. the amino acid sequence of the LCDR1 is shown in SEQ ID NO.18;
v. the amino acid sequence of the LCDR2 is shown in SEQ ID NO.19;
vi. the amino acid sequence of the LCDR3 is shown in SEQ ID NO.20;
   or, a variant with no more than two amino acid variations in each CDR region in single or multiple CDRs of the six CDR regions of above i~vi;
   wherein the amino acid variation is an addition, deletion or substitution of amino acids, preferably, the amino acid variation is a conservative amino acid substitution.

Preferably, the binding K_{D} of the antibody or its antigen-binding fragment to SEMG1 or SEMG2 is <2×10⁻⁸.

More preferably, said K_{D}<2×10⁻⁸, <1×10⁻⁸, <9×10⁻⁹, <8×10⁻⁹, <7×10⁻⁹, <6×10⁻⁹, < 5×10⁻⁹, <4×10⁻⁹, <3×10⁻⁹, <2×10⁻⁹, <1×10⁻⁹, <1×10⁻¹⁰.

In some embodiments, the antibody or antigen-binding fragment comprises a heavy chain variable region and a light chain variable region, the sequences of which are selected from the group consisting of:
a) the amino acid sequence of the heavy chain variable region comprises an amino acid sequence selected from the group consisting of SEQ ID NO: 13 or a sequence having at least 70%, 80%, 90%, 95% or 99% sequence identity to the sequence in the group;
b) the amino acid sequence of the light chain variable region comprises an amino acid sequence selected from the group consisting of SEQ ID NO: 14 or a sequence having at least 70%, 80%, 90%, 95% or 99% sequence identity to the sequence in the group.

Conservative substitution refers to the substitution of one amino acid by another amino acid within the same class, such as one acidic amino acid substituted by another acidic amino acid, one basic amino acid substituted by another basic amino acid, or one neutral amino acid substituted by another neutral amino acid. Exemplary amino acid substitutions are listed in the following table:

### Exemplary amino acid substitutions

| Original residue | Exemplary substitutions | Preferred substitution |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu, Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

In preferred embodiments, the amino acid variations described in the present invention occur in regions outside the CDRs (e.g., in FRs). More preferably, the amino acid variations described in the present invention occur in the Fc region. In some embodiments, provided are anti-SEMG1 protein antibodies comprising an Fc domain comprising one or more mutations that enhance or weaken the binding of the antibody to an FcRn receptor, e.g., at acidic pH as compared to neutral pH. Non-limiting examples of such Fc modifications include, for example, modifications at position 250 (e.g., E or Q), positions 250 and 428 (e.g., L or F), positions 252 (e.g., L/Y/F/W or T), position 254 (e.g., S or T) and position 256 (e.g., S/R/Q/E/D or T); or modifications at position 428 and/or 433 (e.g., H/L/R/S/P/Q or K) and/or 434 (e.g., A, W, H, F or Y [N434A, N434W, N434H, N434F or N434Y]); or modifications at position 250 and/or 428 modification; or modifications at position 307 or 308 (e.g., 308F, V308F) and position 434. In one embodiment, the modifications include modifications at positions 428L (e.g., M428L) and 434S (e.g., N434S); modifications at positions 428L, 259I (e.g., V259I) and 308F (e.g., V308F); modifications at positions 433K (e.g., H433K) and 434 (e.g., 434Y); modifications at positions 252, 254, and 256 (e.g., 252Y, 254T, and 256E); modifications at positions 250Q and 428L (e.g., T250Q and M428L); and modifications at positions 307 and/or 308 (e.g., 308F or 308P). In yet another embodiment, the modification comprises modifications at positions 265A (e.g., D265A) and/or 297A (e.g., N297A).

For example, the present invention includes an anti-SEMG1 protein antibody comprising an Fc domain containing one pair (group) or multiple pairs (groups) of mutations selected from: 252Y, 254T and 256E (e.g., M252Y, S254T and T256E); 428L and 434S (e.g., M428L and N434S); 257I and 311I (e.g., P257I and Q311I); 257I and 434H (e.g., P257I and N434H); 376V and 434H (e.g., D376V and N434H); 307A, 380A and 434A (e.g., T307A, E380A and N434A); and 433K and 434F (e.g., H433K and N434F). In one embodiment, the invention includes an anti-SEMG1 protein antibody comprising an Fc domain containing the S108P mutation in the IgG4 hinge region to promote dimer stabilization. Any possible combination of the foregoing Fc domain mutations and other mutations within the antibody variable domains disclosed herein is within the scope of the present invention.

In other embodiments, the SEMG1 or SEMG2 protein antibodies provided herein may be altered to increase or decrease their degree of glycosylation. Addition or deletion of glycosylation sites on SEMG1 or SEMG2 protein antibodies can be conveniently accomplished by altering the amino acid sequence to create or remove one or more glycosylation sites. When the SEMG1 protein antibody contains an Fc region, the carbohydrate linked to the Fc region can be altered. In some applications, modifications to remove unwanted glycosylation sites may be useful, such as removal of fucose moieties to improve antibody-dependent cellular cytotoxicity (ADCC) function (*See* Shield et al. (2002) JBC277:26733). In other applications, galactosylation modifications can be made to modulate complement dependent cytotoxicity (CDC). In some embodiments, one or more amino acid modifications can be introduced into the Fc region of the coronavirus S protein antibody provided herein to generate variants of the Fc region, so as to enhance efficacy of, for example, the coronavirus S protein antibody of the present invention in the prevention and/or treatment of coronavirus infection.

In some embodiments, the antibody is an IgG1, IgG2, IgG3 or IgG4 antibody; preferably, it is an IgG1 or IgG4 antibody; more preferably, it is a human or murine IgG1 or IgG4 antibody.

In some embodiments, the antibody may further comprise a conjugation moiety linked to the polypeptide selected from one or more of the group consisting of radionuclides, drugs, toxins, cytokines, enzymes, fluoresceins (luciferins), carrier proteins, lipids, and biotins, wherein the polypeptide or antibody and the conjugation moiety is optionally connected through a linker, preferably the linker is a peptide or a polypeptide.

It is understood in the art that, without changing the core functional region of the antibody, the antibody can be prepared or selected from monoclonal antibody, polyclonal antibody, antiserum, chimeric antibody, humanized antibody and human antibody; more preferably, the antibody may be prepared as or selected from multispecific antibody, single chain Fv (scFv), single chain antibody, anti-idiotypic (anti-Id) antibody, diabody, minibody, nanobody, single domain antibody, Fab fragment, F(ab') fragments, disulfide-linked bispecific Fv (sdFv) and intrabody.

The present invention also provides an isolated polynucleotide encoding the above-mentioned antibody or antigen-binding fragment.

The present invention also provides a recombinant vector, comprising the above-mentioned polynucleotide, and optional regulatory sequences; preferably, the recombinant vector is a cloning vector or an expression vector; more preferably, the regulatory sequence is selected from a leader sequence, a polyadenylation sequence, a propeptide sequence, a promoter, a signal sequence, a transcription terminator, or any combination thereof.

The present invention also provides a host cell, characterized in comprising the above-mentioned recombinant vector; preferably, the host cell is a prokaryotic cell or a eukaryotic cell.

The present invention also provides a pharmaceutical composition, characterized in comprising one or more of the above-mentioned antibody or antigen-binding fragment, polynucleotide, recombinant vector, host cell and pharmaceutical composition; preferably, the composition also comprises a pharmaceutically acceptable carrier or adjuvant.

The present invention also provides a kit, characterized in comprising one or more of the above-mentioned antibody or antigen-binding fragment, polynucleotide, recombinant vector, host cell and pharmaceutical composition, and contained in a suitable container.

### Antibody Preparation Method of the Present Invention

Anti-SEMG1 monoclonal antibodies (mAbs) and human sequence antibodies of the invention can be produced by a variety of techniques, including conventional monoclonal antibody methodology, such as the standard somatic cell hybridization technique of Kohler and Milstein (1975) Nature 256:495. Any technique for the production of monoclonal antibodies may be used, such as viral or oncogenic transformation of B lymphocytes. One animal system for preparing hybridomas is the murine system. Production of hybridomas in mice is a well-established method. Immunization protocols and techniques for isolating immunized splenocytes for fusion are known in the art. In some embodiments, the present invention is obtained by immunizing mice multiple times with recombinant SEMG1 protein.

### Activity Assays for Antibodies of the Present Invention

The SEMG1 protein antibodies provided herein can be identified, screened or characterized for their physical/chemical properties and/or biological activity by various assays known in the art. In one aspect, the SEMG1 protein antibody of the present invention is tested for its antigen-binding activity, for example, by known methods such as ELISA, Western blotting and the like. Binding to SEMG1 or SEMG2 proteins can be assayed using methods known in the art, exemplary methods are disclosed herein. In some embodiments, the binding of the SEMG1 protein antibody of the present invention to SEMG1 or SEMG2 protein is determined using bio-layer interferometry, and the binding of SEMG1 or SEMG2 to CD27, LILRB2, LILRB4 or TIGIT is further analyzed by ELISA experiments.

### 5. Pharmaceutical Composition of the Present Invention

In some embodiments, the present invention provides compositions comprising any of the compounds, SEMG1 or SEMG2 protein inhibitors, antagonists, antibodies, antigenic peptides, proteins and the like described herein, preferably the compositions are pharmaceutical compositions.

In one embodiment, the composition comprises an antibody or antigen-binding fragment thereof, or an immunoconjugate, or a bispecific molecule of the present invention, and a pharmaceutically acceptable carrier.

In one embodiment, the pharmaceutical composition comprises the SEMG1 or SEMG2 protein antibody of the present invention in combination with one or more other therapeutic agents.

In some embodiments, the pharmaceutical composition or pharmaceutical preparation of the present invention comprises suitable pharmaceutical adjuvants, such as pharmaceutical carriers and pharmaceutical excipients known in the art, including buffers.

As used herein, "pharmaceutically acceptable carrier" includes any and all solvents, dispersion media, isotonic and absorption delaying agents, and the like that are physiologically compatible. Pharmaceutical carriers suitable for use in the present invention can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable excipients include starch, dextrose, lactose, sucrose, gelatin, malt, rice, flour, chalk, silica gel, sodium stearate, glyceryl monostearate, talc, sodium chloride, dried skim milk, glycerin, propylene, glycol, water, ethanol and the like. For the application of excipients and their uses, see also "Handbook of Pharmaceutical Excipients", Fifth Edition, R. C. Rowe, P.J. Seskey and S.C. Owen, Pharmaceutical Press, London, Chicago. The composition, if desired, can also comprise minor amounts of wetting or emulsifying agents, or pH buffering agents. These compositions may take the form of solutions, suspensions, emulsions, tablets, pills, capsules, powders, sustained release formulations and the like. Oral formulations can comprise standard pharmaceutical carriers and/or excipients, such as pharmaceutical grade mannitol, lactose, starch, magnesium stearate, saccharine.

It can be obtained by combining SEMG1 protein antibody of the present invention with required purity, with one or more optional pharmaceutical adjuvants (Remington's Pharmaceutical Sciences, 16th Edition, Osol, A. ed. (1980)), to prepare pharmaceutical formulations comprising the SEMG1 or SEMG2 protein antibodies described herein, preferably in the form of lyophilized formulations or aqueous solutions.

The pharmaceutical compositions or formulations of the present invention may also contain more than one active ingredient as required for the particular indication being treated, preferably those with complementary activities that do not adversely affect each other. For example, it is desirable to also provide other active ingredients, including but not limited to CTLA4 and PD-1 inhibitors and the like. The active ingredients are suitably present in combination in amounts effective for the intended use.

### 6. Preventive or Therapeutic Use of the Present Invention

### Method for Prevention or Treatment

The invention provides a method for preventing cancer associated with SEMG1 or SMEG2 in a subject, comprising administering to the subject an inhibitor, antibody or pharmaceutical composition of the present invention. Administration of prophylactic agents can be administered prior to the manifestation of symptomatic features in order to prevent the onset of the disease, or alternatively delay the progression of the disease.

Preferably, the subject has received or is receiving or will receive additional anticancer therapy;
More preferably, the additional anticancer therapy comprises surgery, radiotherapy, chemotherapy, immunotherapy or hormone therapy.

The present invention provides a method for treating a cancer associated with SEMG1 or SMEG2 in a subject, comprising administering to the subject an inhibitor, antibody or pharmaceutical composition of the present invention. The administration of the therapeutic agent can be administered after the manifestation of symptoms.

Preferably, the subject has received or is receiving or will receive additional anticancer therapy;
More preferably, the additional anticancer therapy comprises surgery, radiotherapy, chemotherapy, immunotherapy or hormone therapy.

Cancer associated with SEMG1 or SMEG2 include but not limited to various cancers, such as common colorectal cancer, lung cancer, melanoma, lymphoma, liver cancer, head and neck cancer, gastric cancer, kidney cancer, bladder cancer, prostate cancer, testicular cancer, endometrium cancer, breast cancer, and ovarian cancer, etc.

The treatment method provided by the present invention can also be contacting the subject's immune cells and tumor cells with an effective amount of the antibody or antigen-binding fragment, polynucleotide, recombinant vector, host cell and pharmaceutical composition of the present invention; optionally, before contacting an effective amount of the compound with the subject's immune cells and/or tumor cells, detecting the expression of SEMG1 or SEMG2 in the tumor cells;
Preferably, the immune cells are lymphocytes;
More preferably, the lymphocytes are T lymphocytes.

### Prophylactic or Therapeutic Use

It can be understood that the use of the inhibitor, antibody or antigen-binding fragment, polynucleotide, recombinant vector, host cell and pharmaceutical composition of the present invention may at least include the following preventive and therapeutic uses or the uses in the preparation of corresponding medicaments:
a) use in agonizing or antagonizing the interaction of SEMG1 or SEMG2 with CD27, LILRB2, LILRB4 or TIGIT; preferably SEMG1 or SEMG2 is expressed in tumor cells, and CD27, LILRB2, LILRB4 or TIGIT is expressed in immune cells;
b) use in the preparation of a product for agonizing or antagonizing the interaction of SEMG1 or SEMG2 with CD27, LILRB2, LILRB4 or TIGIT; preferably SEMG1 or SEMG2 is expressed in tumor cells, and CD27, LILRB2, LILRB4 or TIGIT is expressed in immune cells;
c) use in the prevention or treatment of tumors;
d) use in the preparation of medicament for the prevention or treatment of tumors;
e) use in modulating the immune response elicited against tumors;
f) use in the preparation of medicament for regulating immune responses against tumors;
g) use in inhibiting tumor cell growth *in vitro* and *in vivo;*
h) use in the preparation of reagents for inhibiting tumor cell growth *in vitro* and *in vivo.*

### 7. Diagnostic Use of the Present Invention

The term "companion diagnostic" generally refers to the provision of information about a patient's response to a specific therapeutic agent, helping to identify patient populations who could benefit from a therapeutic product, thereby improving treatment outcomes and reducing healthcare costs. In addition, companion diagnostics can help identifying patient populations most likely to respond to therapeutic drugs.

The present invention confirms that SEMG1 or SEMG2 protein can be used as a therapeutic target for cancers associated with SEMG1 or SMEG2, that is, when SEMG1 or SEMG2 protein is expressed in tumor cells, the tumor can be effectively inhibited by introducing SEMG1 or SEMG2 protein inhibitors (such as antibodies). Therefore, during the treatment of corresponding tumor patients (including before and after treatment), the SEMG1 or SEMG2 protein expression of tumor cells can be detected to determine the patient population most likely to respond to the therapeutic drug. Therefore, the present invention provides a companion diagnosis method, which is accomplished by detecting the SEMG1 or SEMG2 protein expression or function of the subject's tumor cells before/after immunotherapy.

### 8. Kit of the Present Invention

Kits comprising an antibody or antibody composition (e.g., a human antibody, bispecific or multispecific molecule, or immunoconjugate) of the invention and instructions for use are also within the scope of the invention. The kit may further comprise at least one additional pharmaceutical agent, or one or more additional antibodies, and the like. The kit typically includes a label indicating the intended use of the kit's contents. The term label includes any written or recorded material provided on or with the kit or otherwise accompanying the kit.

In addition, based on the diagnostic use of the present invention, the kit of the present invention may also include components capable of detecting the expression or activity of SEMG1 or SEMG2 protein in tumor cells. Therefore, in addition to the above-mentioned antibody composition, the kit components of the present invention may also include primers, probes and the like, or other materials capable of detecting protein expression or activity, which are not limited herein.

### Brief Description of the Drawings

In order to more clearly illustrate the specific embodiments of the present invention or the technical solutions in prior art, the following will briefly introduce the accompanying drawings that need to be used in the description of specific embodiments or the prior art. It will be apparent that the accompanying drawings in the following description are some of the embodiments of the present invention, and the skilled person in the art can obtain other drawings based on these drawings without any creative work.

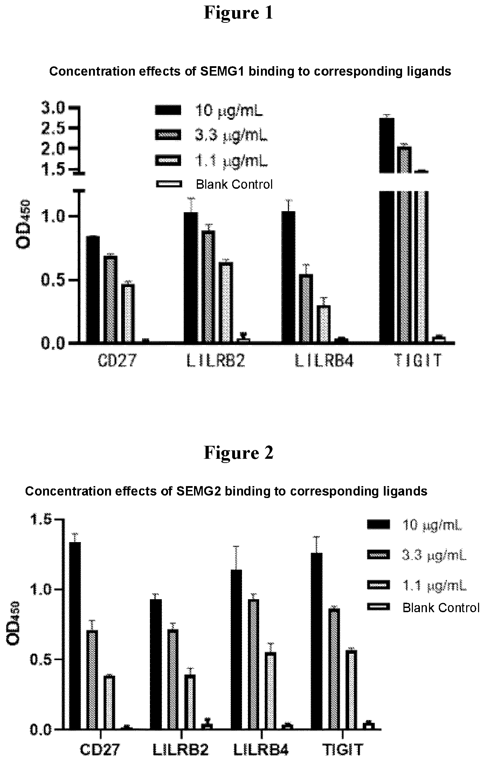

Figure 1. Concentration effect of SEMG1 binding to corresponding receptor proteins. The ability of different concentrations of SEMG1 to bind to CD27 (2 µg/mL), LILRB2 (2 µg/mL), TIGIT (3 µg/mL) and LILRB4 (2 µg/mL) increased with the concentration of SEMG1, indicating that SEMG1 has the capability to bind specifically to receptor proteins CD27, LILRB2, TIGIT or LILRB4. The OD₄₅₀ readings of each positive group in the figure of examples have been subtracted by the OD₄₅₀ readings after color development of the negative control which was blank-coated, blocked and then added with the corresponding ligand protein.
Figure 2. Concentration effect of SEMG2 binding to corresponding receptor proteins. The ability of different concentrations of SEMG2 to bind 2 µg/mL of CD27 (2 µg/mL), LILRB2 (2 µg/mL), TIGIT (3 µg/mL) and LILRB4 (2 µg/mL) increased with the concentration of SEMG2, indicating that SEMG2 has the capability to specifically bind to receptor proteins CD27, LILRB2, TIGIT or LILRB4. The OD₄₅₀ readings of each positive group in the figure have been subtracted by the OD₄₅₀ readings after color development of the negative control which was blank-coated, blocked and then added with the corresponding ligand protein.

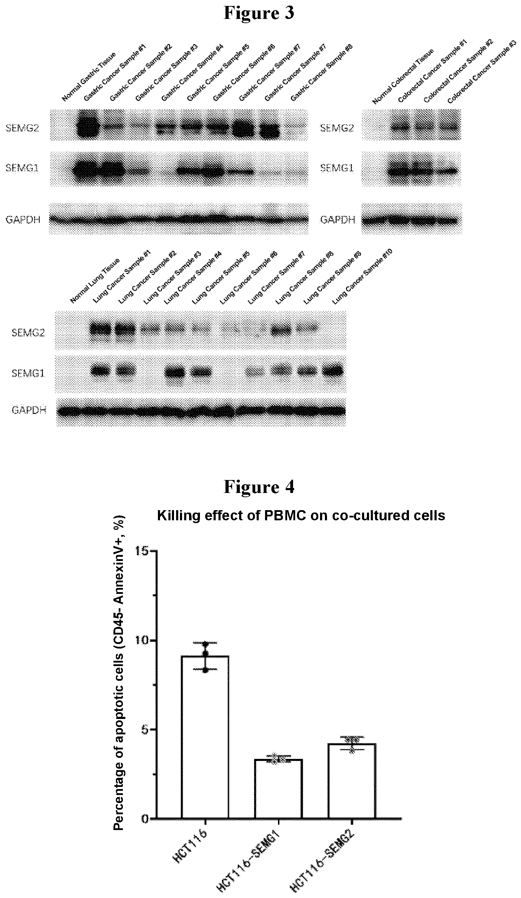

Figure 3. Expression identification of SEMG1 and SMEG2 in tumor tissues. Protein suspension prepared by lysing gastric wall and gastric tumor tissue extracts, gastric wall and gastric tumor tissue extracts, colorectum and colorectal tissue extracts from different pathological tissues of tumor patients.
Figure 4. Inhibitory effect of overexpression of SEMG1 or SEMG2 on tumor cell killing by PBMCs. After co-incubating PBMC cells with tumor cells or tumor cells overexpressing SEMG1 or SEMG2, respectively, the percentage of apoptotic cells was determined. The vertical axis is the percentage of apoptotic tumor cells; the horizontal axis is different experimental treatment conditions, that is, different cells added. The results show that overexpression of SEMG1 or SEMG2 can significantly inhibit the killing effect of binding activated PBMC on tumor cells.

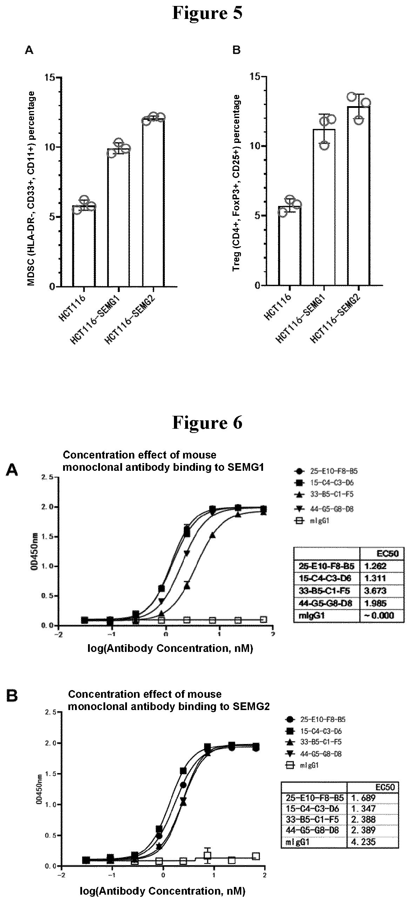

Figure 5. Induction effect of MDSC and Treg by HCT116 cell line overexpressing SEMG1 and SEMG2. Compared with HCT116 cells, co-culturing HCT116 cells overexpressing SEMG1 and SEMG2 with human peripheral blood mononuclear cells (PBMC) can significantly increase the ratio of MDSC cells (HLA-DR-, CD33+, CD11+) or Treg cells (CD4+FoxP3+CD25+), indicating SEMG1 and SEMG2 realize the immune escape of tumor cells by inducing the transformation of MDSC and Treg.
Figure 6. Concentration effect of mouse hybridoma antibody binding to SEMG1 and SEMG2. The vertical axis is the OD₄₅₀ absorbance value as the reading of the ELISA experiment, showing the binding level of 1 µg/mL SEMG1 (A) or SEMG2 (B) immobilized on the microwell plate and the added mouse hybridoma monoclonal antibody; the horizontal axis shows different concentrations of incubated antibody. Different curves represent antibodies produced by different clones, and the irrelevant murine monoclonal antibody mIgG1 serves as a negative control. The EC50 values of the corresponding antibodies are also indicated in the figure. Fitted curve is one representative result based on three independent biological experiments (error bars represent standard deviation).

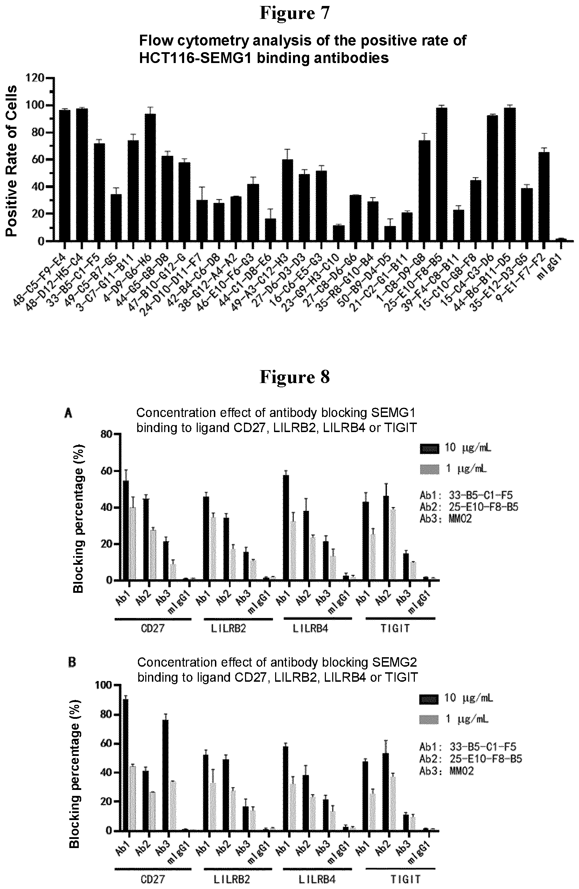

Figure 7. The binding positive rate of mouse hybridoma monoclonal antibody binding to HCT116 cells overexpressing SEMG1 detected by flow cytometry. The results show that cells overexpressing SEMG1 have different positive rates when binding to hybridoma monoclonal antibody (5 µg/mL) of different clones, indicating the diversity of antibodies and the ability of antibodies binding to antigens expressed on the surface of tumor cells.
Figure 8. Blocking effect of mouse hybridoma antibodies SEMG1 or SEMG2 binding to the corresponding proteins. The figure shows the ELISA binding test of two monoclonal antibodies 33-B5-C1-F5 and 25-E10-F8-B5 to block the binding of 2 µg/mL SEMG1 protein (A) or 22 µg/mL SEMG2 protein (B) at two concentrations to the corresponding proteins: 2 µg/mL CD27, 2 µg/mL LILRB2, 3 µg/mL TIGIT and 2 µg/mL LILRB4, and the blocking percentage was calculated by comparing with OD₄₅₀ values of ELISA of the coated microwells with the ligand proteins and PBS added. The blocking percentage represents the blocking level, and the higher the blocking percentage, the better the effect of blocking the binding of the target protein to the ligand.

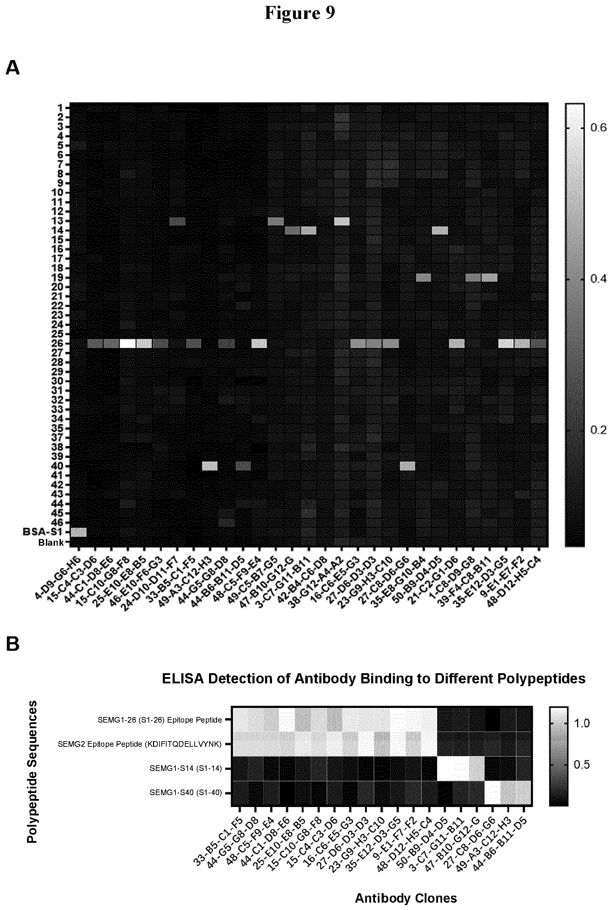

Figure 9. Heat map of identification of antibody binding to SEMG1 and SEMG2 polypeptides. (A) After coating the 96-well microplate with polypeptide (table) and SEMG1 protein (positive control), 2.5 µg/mL of corresponding monoclonal antibodies produced by 29 hybridoma cell lines are added respectively, and after conventional ELISA, the OD value was detected with rabbit anti-mouse HRP secondary antibody. Antibody-binding polypeptides whose corresponding values are more than 3 times higher than the background value, are considered to be polypeptides that antibody specifically binds to. All wells coated with full-length SEMG1 had the highest machine-readable readings. Graphpad prism was used to draw a heat map, the polypeptide sequence of the peptide is marked as the vertical axis, and the antibody reaction is marked as the horizontal axis, and the intersection of each vertical and horizontal axis is the response value of a polypeptide corresponding to an antibody. It can be seen from the heat map that the isolated antibodies mainly bind to BSA-S1, S1-13, S1-14, S1-19, S1-26, and S1-40. Among them, 10 monoclonal antibodies bind to the S1-26 polypeptide, indicating that the sequence of this polypeptide is the main antigenic determinant for producing SEMG1 monoclonal antibody, and it is a key segment for SEMG1 to stimulate the body to produce antibodies. (B) SEMG1-26 (S1-26) epitope peptide, SEMG2 corresponding epitope peptide (KDIFITQDELLVYNK), SEMG1-S14 (S1-14), SEMG1-S40 (S1-40) polypeptide was used to coat ELISA plate, and then the different antibody clones shown in the figure were used for binding and detection. The results showed that the antibody capable of binding to SEMG1-26 (S1-26) also had significant binding to the corresponding epitope peptide of SEMG2 (i.e., the amino acid sequence highly similar to regional sequence KDIFITQDELLVYNK of SEMG1). This suggests that the dominant epitopes of SEMG1 and SEMG2 bind the same antibody clone.

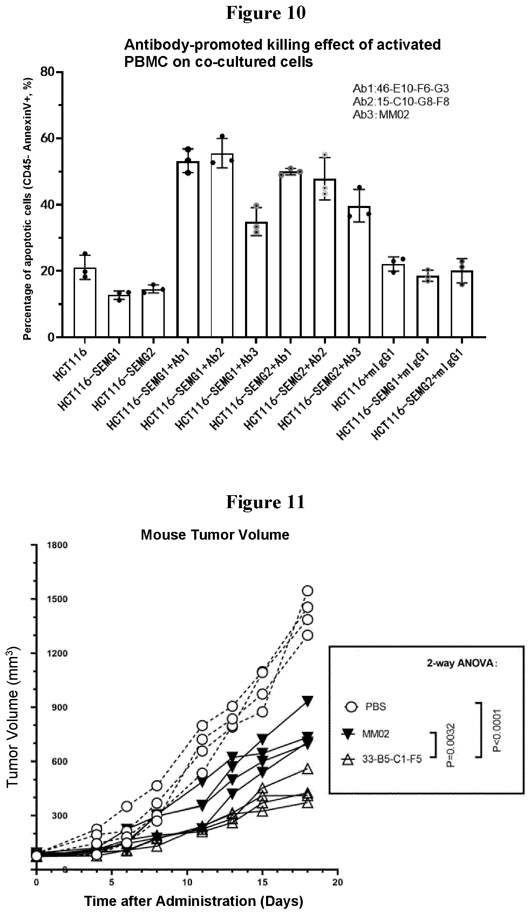

Figure 10. Antibodies against SEMG1 and SEMG2 promote PBMC killing effect on tumor cell lines. Activated PBMC human peripheral blood mononuclear cells were co-cultured with HCT116 and HCT116 cells overexpressing SEMG1 and SEMG2, and different antibodies were added at the same time: irrelevant mouse IgG, MM02 mouse anti-SMEG2 antibody, and SEMG1 and SEMG2-binding mouse monoclonal antibodies 33-B5-C1-F5 and 25-E10-F8-B5. The vertical axis is the percentage of apoptotic tumor cells; the horizontal axis is the different experimental treatment conditions, that is, different cells and antibodies added. The results show that overexpression of SEMG1 or SEMG2 can inhibit the killing effect of activated PBMC on tumor cells to a certain extent, while the mouse monoclonal antibodies 33-B5-C1-F5 and 25-E10-F8- B5, which simultaneous bind to SEMG1 and SEMG2, significantly promoted the killing of tumors by T cells, and was superior to monoclonal antibody MM02 of SEMG2, while the control of irrelevant mIgG did not show this function. This indicates that the expression of SEMG1 and SEMG2 is very important for the immune escape function of tumor cells, and antibodies against SEMG1 and SEMG2 play an important role in the immune system killing tumor cells.
Figure 11. Inhibition of tumorigenesis by antibodies in a mouse *in vivo* model. After NPSG mice were inoculated with PBMCs, they were inoculated with human breast cancer cells MCF7, which naturally express SEMG1 and SEMG2, to form tumors on the 3rd day, the mice were grouped, and the tumor-bearing mice were treated with the SEMG1 monoclonal antibody 33-B5-C1-F5, the SEMG2 monoclonal antibody MM02, and PBS as tumor inhibitors, respectively. The graphs show that the monoclonal antibody 33-B5-C1-F5 inhibited tumors significantly stronger than the PBS control group, and the therapeutic effect was better than that of the SEMG2 monoclonal antibody MM02, indicating the therapeutic effect of SEMG1 monoclonal antibody on the tumors expressing SEMG1 and SEMG2.

### Examples

The experimental methods in the following examples are conventional methods unless otherwise specified. The invention will be further understood with reference to the following non-limiting experimental examples. These examples are for illustrative purposes only and do not limit the scope of the claims presented herein.

### Example 1: Detection of binding of SEMG1 or SEMG2 to receptor proteins LILRB2, TIGIT or LILRB4 proteins

The binding and binding strength of SEMG1 or SEMG2 to receptor proteins CD27, LILRB2, TIGIT or LILRB4 were analyzed by ELISA experiment. Specifically, a special plate for ELISA (Costar, ME, USA) was used. First, the ELISA coating solution (Solarbio, Beijing, China) containing SEMG1 or SEMG2 recombinant protein with final concentrations of 10 µg/mL, 3.3 µg/mL, and 1.1 µg/mL was used to coat the plate in duplicate, and 100 µL of coating solution without protein was used as negative control. The plate was coated overnight at 4°C. After washing with PBST, 100 µL of 5% skim milk dissolved in PBS (Sangon, Shanghai, China) was used to block, and then the plate was blocked at 37°C for 90 minutes in an incubator. After washing with PBST, incubation and binding were conducted in PBS solution with the corresponding concentration of the extracellular region protein fragments (ACROBiosystems or SinoBiological, Beijing, China) of receptor proteins (CD27, LILRB2, TIGIT, or LILRB4, as well as SIRP as a negative control, wherein the SEMG2 interacting group of with CD27 was used as a positive control), which were labeled with an hFc tag (P-Fc), and then the plate was incubated at 37°C for 60 minutes. After washing with PBST, the HRP-labeled specific anti-human Fc fragment antibody (Abcam, MA, USA) diluted in PBS was added to incubate at 37°C for 30 minutes. After washing with PBST, color developing solution (Sangon, Shanghai, China) was used for color development, 100 µL per well, and the plate was placed in an incubator for 5-30 minutes, and after adding 50 µL of stop solution (Sangon, Shanghai, China), the plate was placed in a microplate reader (Thermo Fisher, MA, USA) for chromogenic reading at 450 nm.

The results showed that SEMG1 can bind to LILRB2, TIGIT or LILRB4, which proved that LILRB2, TIGIT or LILRB4 are the binding receptors of SEMG1 or SEMG2. The concentration gradient effect of SEMG1 binding to LILRB2, TIGIT or LILRB4 is shown in Figure 1. The concentration gradient effect of SEMG2 binding to LILRB2, TIGIT or LILRB4 is shown in Figure 2.

### Example 2: Detection of the expression of SEMG1 and SEMG2 in tumor tissues of different origins

In order to analyze the distribution of SEMG1 and SEMG2 in tumor tissues, the protein suspensions prepared by lysing the gastric wall and gastric tumor tissue extracts, gastric wall and gastric tumor tissue extracts, colorectum and colorectal tissue extracts of tumor patients (SigBio, Shanghai, China) were used to detect SEMG1 and SEMG1 protein expression, and the lysed protein suspension of corresponding tissue extracts from normal people was used as control. Specifically, a 10% PAGE gel (Epizyme, Shanghai, China) was prepared on a gel plate (Bio-Rad, CA, USA) according to the instructions, and the prepared gel was placed in an electrophoresis tank (Bio-Rad, CA, USA). A power supply (Bio-Rad, CA, USA) was connected to allow the bands to run through the stacking gel at a constant voltage of 80 volts and through the separating gel at a constant voltage of 120 volts. When the bands reached the bottom of the separating gel, the membrane was transferred using wet method in a transfer tank (Bio-Rad, CA, USA) with a constant current of 350 mA for 90 minutes. After the membrane transfer was completed, the membrane was sheared according to the mass size of SEMG1 and GAPDH proteins. Blocked with fast blocking solution (EpiZyme, Shanghai, China) for ten minutes, and incubated the corresponding bands with SEMG1 and SEMG2 co-reactive antibody (preparation method as described below) and GAPDH antibody (Consun, Shanghai, China) respectively, overnight at 4°C. After washing with TBST the next day, they were incubated with specific anti-mouse secondary antibody or anti-rabbit secondary antibody (Conson, Shanghai, China) diluted with 5% skim milk powder (Sangon, Shanghai, China) dissolved in TBS for one hour at room temperature. After washing with TBST, they were placed in a mixed luminescence solution (ShareBio, Shanghai, China) for one minute, and exposed in a gel imager (Bio-Rad, CA, USA). The analysis results of 9 gastric cancer samples, 3 colorectal cancer samples and 10 lung cancer samples are shown in Figure 3. The results showed that SEMG1 and SEMG2 were co-expressed to varying degrees in gastric cancer tissue samples and colorectal cancer samples; and in lung cancer samples, SEMG1 and SEMG2 were co-expressed in 6 samples, 1 sample only expressed SEMG1, and 2 samples only expressed SEMG2. Both SEMG1 and SEMG2 were expressed in 85.7% of all samples analyzed. However, there is no expression of SEMG1 and SEMG2 in gastric tissue, colorectal tissue and lung tissue of normal people. The above results indicate that the expression of SEMG1 and SEMG2 can be used as markers for tumor therapy for tumor diagnosis and as specific therapeutic targets.

### Example 3: Tumor cells overexpressing SEMG1 can significantly reduce the killing of human peripheral blood mononuclear cells to tumor cells

The revived PBMC and HCT116 or overexpression cells were counted, and the cell number was adjusted to 1×10⁶/mL, respectively, and 100 µL of each was added to a 96-well plate (Thermo Fisher, MA, USA), and incubated in an incubator for 6 hours. The 96-well plate was taken out, the cells in each well were placed in EP tubes (Axygen, CA, USA), centrifuged at 400 rcf for 5 minutes, and the supernatant was discarded. The washed cells were resuspended with 500 µL of cell staining buffer (Invitrogen, CA, USA), and the centrifugation and washing were repeated. The CD45-APC antibody (Invitrogen, CA, USA) was diluted 1:20 with cell staining buffer, and 200 µL of the mixture was added to each EP tube to resuspend, and incubated at room temperature for 30 minutes. After centrifugation at 400 rcf for 5 minutes, the supernatant was discarded, and the cells were resuspended and washed with 1 mL of binding buffer (MeilunBio, Shanghai, China); the centrifugation and washing were repeated. Each experiment and control group was set, and 100 µL of binding buffer, 5 µL of Annexin V-FITC (MeilunBio, Shanghai, China) and 10 µL of PI (MeilunBio, Shanghai, China) were added separately according to the conditions, and the plate was incubated at room temperature for 15 minutes, and then 400 µL of binding buffer was added respectively, the solutions were transferred to flow tubes (Falcon, NY, USA) and tested on the machine (Miltenyi, Cologne, Germany), the results are shown in Figure 4, indicating that SEMG1 overexpression inhibits killing effect of PBMC on tumor cells.

### Example 4: SEMG1 induces transformation of PBMC myeloid-derived suppressor cells (MDSC) and regulatory T cells (Treg)

In order to elucidate the effect of SEMG1 on Treg/MDSC, cells were first prepared with 1.5*10⁵ PBMC per well, and different experimental groups cultured for 24h (for detecting the ratio of Treg) or 3 days (for detecting the ratio of MDSC) were performed. Phenotype CD4+CD25+Foxp3+, HLA-DR-CD11b+CD33+ ratio and count were analyzed by flow cytometry.

The results of the analysis showed that co-culture of SEMG1-expressing HCT116 cells with human peripheral blood mononuclear cells increased the ratio of MDSC cells (HLA-DR-CD11b+CD33+) and Treg (CD4+CD25+Foxp3+) cells as compared to normal HCT116 cells, and the result is shown in Figure 5.

### Example 5: Preparation of mouse hybridoma monoclonal antibody

The SEMG1 protein was expressed and purified with the *E. coli* system, and the purity reached 92% by tested, and the ELISA experiment was used to verify that the SEMG1 protein has the activity of binding to receptors and LILRB2. 10 mice were immunized with the protein, and multiple immunizations were carried out to enhance the effect: (1) initial immunization, antigen 50 µg/mouse, with Freund's complete adjuvant, subcutaneous injection in multiple points, with an interval of 3 weeks; (2) second immunization, dosage and route as above, with Freund's incomplete adjuvant, with an interval of 3 weeks; (3) third immunization, the dosage and route as above, and at the same time, the SEMG2 protein is used for cross-immunization, without adjuvant, intraperitoneal injection, with an interval of 3 weeks; (4) booster immunization, dose 50 µg, intraperitoneal injection. Blood was collected 3 days after the last injection to measure its potency, to detect the immune effect. The mice whose immune titer reached the target were sacrificed, and the spleen and lymph nodes were collected for fusion to generate hybridomas. Hybridomas were grown in a 96-well tissue culture plate, and supernatants from individual wells were screened by ELISA to identify positive binders of the target antigen SEMG1 and SEMG2 proteins. After subcloning, the binding of monoclonal antibody to SEMG1 and SEMG2 was detected by ELISA.

### Example 6: Construction of HCT116-SEMG1 cell line overexpressing SEMG1

SEMG1 and SEMG2 were cloned into pCDNA3.1 vector and transfected with DH5α to prepare sterile plasmids for transfection. 5-10×10⁶ cells were inoculated in 6-well cell plates, cultured overnight with medium containing 10% calf serum, and used for transfection when about 60%-80% confluent. The supernatant in the culture plate was discarded, and the cells were washed three times with no-resistance and serum-free culture medium, 800 µL of medium was added to each well, and then rewarmed FuGENE HD transfection reagent was added, 90 µL of medium and 4 µL of plasmid with a concentration of 500 ng/µL were added into the 100 µL reaction system, then mixed with a vortex mixer; then 6 µL of FuGENE HD transfection reagent was added, and the mixture was immediately shook to be mixed well, and left to stand at room temperature for 7-12 minutes, and then the mixture was added dropwise to the cell plate well evenly, and mixed well by pipetting or vortexing. The plate was placed in a 37°C incubator for incubation. Add serum-containing medium 2 hours after transfection, so that the final concentration of serum reached 1-4%, and the total amount of supernatant reached 1.5 mL. After the cell plate was cultured in a 37°C incubator for 24-48 hours, 600 µg/mL of G418 screening pressure was added for screening. A control for transfection of the pCDNA3.1 plasmid and a control of empty transfection were also set. When more than 90% of the cells in the control of empty transfection died, the state of the cells in the transfection group was observed, and the medium was changed, and 600 µg/mL G418 medium was continuously used for screening. After the transfected cells proliferated stably, they were maintained with 300 µg/mL G418 medium. Cells were rinsed three times with 4°C pre-cooled PBS, and lysed by adding Western cell lysis buffer containing PMSF for 15 minutes on ice. The cells were then scraped and transferred to a 1.5 mL EP tube, centrifuged at 12,000 rpm for 10 minutes, and the supernatant was taken and added with 5×SDS loading buffer, mixed well, and then placed in a boiling water bath and boiled for 10 minutes. Identification by western blotting was performed after SDS-PAGE.

### Example 7: ELISA and BLI bio-layer interferometry to detect the intensity of mouse hybridoma monoclonal antibody binding to SEMG1 and SEMG2

In order to identify whether the screened monoclonal antibodies with neutralizing effect can bind to SEMG1 and SEMG2 proteins, we coated SEMG2 protein diluted in carbonate buffer (Solarbio, Beijing, China) to a final concentration of 1 µg/mL on a dedicated ELISA plate (costar, ME, USA), and the binding of 1 µg/mL mouse hybridoma antibody to SEMG2 was initially tested. According to the determination outcome, the binding and binding strength between SEMG1 or SEMG2 and the purified mouse monoclonal antibody were further analyzed by ELISA experiment, and an irrelevant mouse monoclonal antibody was taken as a negative control. Specifically, a special plate for ELISA (costar, ME, USA) was used. First, different concentrations of SEMG1 or SEMG2 recombinant proteins were dissolved in 100 µL of carbonate coating buffer (Solarbio, Beijing, China) to 2 µg/mL to coat the plate, and the negative control was coated with 100 µL of coating buffer without protein. The plates were coated overnight at 4°C. The plates were washed with PBST and then blocked with 100 µL of 5% skim milk dissolved in PBS (Sangon, Shanghai, China) for 90 min at 37°C in an incubator. After washing with PBST, the plate was incubated with monoclonal antibodies diluted in 3-fold concentration series starting from 10 µg/mL in PBS at 37°C for 60 minutes. After washing with PBST, HRP-labeled specific goat anti-mouse antibody (Jackson IR, PA, USA) diluted in PBS was used to incubate and bind at 37°C for 30 minutes. After washing with PBST, color developing solution (Sangon, Shanghai, China) was used for color development, 100 µL per well, the plate was placed in an incubator for 5-30 minutes, and adding 50 µL of stop solution (Sangon, Shanghai, China) to stop the reaction, and then the plate was placed in a microplate reader (Thermo Fisher, MA, USA) for chromogenic reading at 450 nm. The concentration-effect curves of antibody binding to the coated proteins were plotted using graphpad prism, and the results showed that monoclonal antibodies that bind well to SEMG1 have good binding ability to SEMG2, as shown in Figure 6.

**Table 1. EC50 values of ELISA detection of antigen antibody binding to SEMG1 and SEMG2**

| | EC50(nM), SEMG1 | EC50(nM), SEMG2 |
|---|---|---|
| 25-E10-F8-B5 | 1.26 | 1.689 |
| 15-C4-C3-D6 | 1.31 | 1.347 |
| 33-B5-C1-F5 | 3.67 | 2.388 |
| 44-G5-G8-D8 | 1.98 | 2.389 |

### Example 8: Binding positivity of SEMG1 murine hybridoma monoclonal antibody binding to HCT116-SEMG1 overexpressing cells detected by flow cytometry analysis

The medium of freshly cultured well-growth HCT116 or overexpressed cells was discarded, and the plate was washed by PBS, then an appropriate amount of 0.25% trypsin was added, shook gently up and down until the cells are about to fall, and complete culture medium was immediately added to neutralize the trypsin, and the cells were placed in a 15 mL centrifuge tube, and centrifuged at 800 rpm for 4 min. The supernatant was discarded, and the cells were resuspended by PBS and centrifuged at 800 rpm for 4 min, and repeated once. The cells were counted and adjusted to an appropriate number respectively. The cells were resuspended with eBioscience Flow Cytometry Staining Buffer (Thermo fisher) evenly and distributed into 1.5 mL EP tubes, and the prepared mouse monoclonal antibody was added at a final concentration of 5 µg/mL to incubate for 1 hour. Then the tubes were centrifuged at 400 rpm for 5 min at 4°C and the supernatant were discarded, and PBS was added to resuspend the cells, and repeated once. The rabbit anti-mouse FITC secondary antibody (Abeam) was diluted 1:400 with eBioscience Flow Cytometry Staining Buffer (Thermo fisher), and after preparation, it was evenly distributed to each reaction tube, and then placed on a shaker at room temperature for 45 min. The tubes were centrifuged at 400 rpm, 4°C for 5min, and the supernatant was discarded, the cells were resuspended by PBS, and 400 µL of binding buffer was added after centrifugation again, the solutions were transferred to flow tubes (Falcon, NY, USA) and tested on the machine (Miltenyi, Cologne, Germany) The results are shown in Figure 7.

### Example 9: Identification of antibody binding to SEMG1 polypeptides and key epitopes

1/3 overlapping peptide sequences were designed, and a peptide library consisting of 45 peptides (GenScript, Nanjing) was synthesized. The synthesized polypeptide sequences are shown in Table 2. Peptide matrix was prepared by using a polypeptide-coated 96-well plate (Costar, Corning), and the selected monoclonal antibody was used as the primary antibody for ELISA binding test, and SEMG1 coating was set as a positive control, and BSA coating was set as a control. The ELISA procedure was performed routinely, as described in Example 2 above. For the coated polypeptide well with OD_{experimental group}/OD_{blank group}>3, it was determined as a positive reaction, which was the corresponding antibody-bound polypeptide sequence. According to the polypeptides that antibodies can bind to, the classification of corresponding antibodies is shown in Table 3.

**Table 2. The sequences of the synthetic polypeptides and the amino acid position of the corresponding SEMG1 protein**

| **Polypeptide No.** | Polypeptide Sequences | **(Corresponding SEMG1 AA position** |
|---|---|---|
| **S1-1** | MKPNIIFYLSLLLIL (Seq ID NO: 1) | 1-15 |
| **S1-2** | LLLILEKQAAVMGQK (Seq ID NO: 2) | 11-25 |
| **S1-3** | VMGQKGGSKGRLPSE (Seq ID NO: 3) | 21-35 |
| **S1-4** | RLPSEFSQFPHGQKG (Seq ID NO: 4) | 31-45 |
| **S1-5** | HGQKGQHYSGQKGKQ (Seq ID NO:5) | 41-55 |
| **S1-6** | QKGKQQTESKGSFSI (Seq ID NO: 6) | 51-65 |
| **S1-7** | GSFSIQYTYHVDAND (Seq ID NO: 7) | 61-75 |
| **S1-8** | VDANDHDQSRKSQQY (Seq ID NO: 8) | 71-85 |
| **S1-9** | KSQQYDLNALHKTTK (Seq ID NO: 9) | 81-95 |
| **S1-10** | HKTTKSQRHLGGSQQ (Seq ID NO: 10) | 91-105 |
| **S1-11** | GGSQQLLHNKQEGRD (Seq ID NO: 11) | 101-115 |
| **S1-12** | QEGRDHDKSKGHFHR (Seq ID NO: 12) | 111-125 |
| **S1-13** | GHFHRVVIHHKGGKA (Seq ID NO: 13) | 121-135 |
| **S1-14** | KCCKAHRGTQNPSQD (Seq ID NO: 14) | 131-145 |
| **S1-15** | NPSQDQGNSPSGKGI (Seq ID NO: 15) | 141-155 |
| **S1-16** | SGKGISSQYSNTEER (Seq ID NO: 16) | 151-165 |
| **S1-17** | NTEERLWVHGLSKEQ (Seq ID NO: 17) | 161-175 |
| **S1-18** | LSKEQTSVSGAQKGR (Seq ID NO: 18) | 171-185 |
| **S1-19** | AQKGRKQGGSQSSYV (Seq ID NO: 19) | 181-195 |
| **S1-20** | QSSYVLQTEELVANK (Seq ID NO: 20) | 191-205 |
| **S1-21** | LVANKQQRETKNSHQ (Seq ID NO: 21) | 201-215 |
| **S1-22** | KNSHQNKGHYQNVVE (Seq ID NO: 22) | 211-225 |
| **S1-23** | QNVVEVREEHSSKVQ (Seq ID NO: 23) | 221-235 |
| **S1-24** | SSKVQTSLCPAHQDK (Seq ID N NO): 24) | 231-245 |
| **S1-25** | AHQDKLQHGSKDIFS (Seq ID NO: 25) | 241-255 |
| **S1-26** | KDIFSTQDELLVYNK (Seq ID NO: 26) | 251-265 |
| **S1-27** | LVYNKNQHQTKNLNQ (Seq ID NO: 27) | 261-275 |
| **S1-28** | KNLNQDQQHGRKANK (Seq ID NO: 28) | 271-285 |
| **S1-29** | RKANKISYQSSSTEE (Seq ID NO: 29) | 281-295 |
| **S1-30** | STEERRLHYGENGV (Seq ID NO: 30) | 292-305 |
| **S1-31** | GENGVQKDVSQSSIY (Seq ID NO: 31) | 301-315 |
| **S1-32** | QSSIYSQTEEKAQGK (Seq ID NO: 32) | 311-325 |
| **S1-33** | KAQGKSQKQITIPSQ (Seq ID NO: 33) | 321-335 |
| **S1-34** | TIPSQEQEHSQKANK (Seq ID NO: 34) | 331-345 |
| **S1-35** | QKANKISYQSSSTEE (Seq ID NO: 35) | 341-355 |
| **S1-37** | GENGVQKDVSQRSIY (Seq ID NO: 36) | 316-375 |
| **S1-38** | QRSIYSQTEKLVANK (Seq ID NO: 37) | 371-385 |
| **S1-39** | LVAGKSQIQAPNPKQ (Seq ID NO: 38) | 381-395 |
| **S1-40** | PNPKQEPWHGENAKG (Seq ID NO: 39) | 391-405 |
| **S1-41** | ENAKGESGQSTNREQ (Seq ID NO: 40) | 401-415 |
| **S1-42** | TNREQDLLSHEQKGR (Seq ID NO: 41) | 411-425 |
| **S1-43** | EQKGRHQHGSHGGLD (Seq ID NO: 42) | 421-435 |
| **S1-44** | HGGLDIVIIEQEDDS (Seq ID NO: 43) | 431-445 |
| **S1-45** | QEDDSDRHLAQHLNN (Seq ID NO:44) | 441-455 |
| **S1-46** | QHLNNDRNPLFT (Seq ID NO: 45) | 451-462 |
| **BSA-S1** | **BSA-SQRSIYSQTEKLVAGKSQIQAPNPKQ** (Seq ID NO: 46) | **370-395** |

**Table 3. Classification of Monoclonal Antibodies Based on Binding Peptides**

| **Peptide** | **Corresponding Antibodies** | **Count** |
|---|---|---|
| S1-13 | 24-D10-D11-F7, 38-G12-A4A2, 49-C5-B7-G5 | 3 |
| S1-14 | 50-B9-D4-D5, 3-C7-G11-B11, 47-B10-G12-G | 3 |
| S1-19 | 35-E8-G10-B4, 39-F4-C8B11, 1-C8-D9-C8 | 3 |
| S 1-26 | 33-B5-C1-F5, 44-G5-G8-D8, 48-C5-F9-E4, 44-C1-D8-E6, 25-E10-E8-B5, 15-C10-G8-F8, 15-C4-C3-D6, 16-C6-E5-G3, 27-D6-D3-D3, 23-G9-H3-C10, 35-E12-D3-G5, 9-E1-F7-F2, 48-D12-H5-C4 | 13 |
| S 1-40 | 27-C8-D6-G6, 49-A3-C12-H3, 44-B6-B11-D5 | 3 |
| BSA-S1 | 4-D9-G6-H6 | 1 |
| Others | 46-E10-F6-G3, 33-B5-C1-F5, 42-B4-C6-D8 | 3 |

### Example 10: ELISA detection of mouse hybridoma monoclonal antibody blocking SEMG1 or SEMG2 binding to receptor CD27, LILRB2, LILRB4 or TIGIT

In order to screen and identify antibodies that can block the function of SEMG1 or SEMG2, the effects of antibodies on the binding of SEMG1 or SEMG2 to its receptor CD27, LILRB2, LILRB4 or TIGIT were analyzed by ELISA experiments. Specifically, the SEMG1 or SEMG2 recombinant protein was coated on a 96-well microplate, and antibodies of different concentrations (0, 1.1, 3.3, 10.0 µg/mL) were added, and the corresponding recombinant receptor (human antibody Fc fragment or biotin) was added, then anti-human secondary antibody coupled with horseradish peroxidase (Jacksonimmuno, USA) or avidin HRP (Thermo Fisher, USA), and finally color developed with TMB substrate, and the absorbance at 450 nm was detected to indicate the binding level of SEMG1 or SEMG2 to receptors CD27, LILRB2, LILRB4 or TIGIT. Percentage of blocking was calculated according to the formula 1 - (OD_{test group} - OD_{blank})/(OD_{positive control} - OD_{blank}).

As shown in Figure 8, the antibodies showed an inhibitory effect on the binding of SEMG1 or SMEG2 to its receptor CD27, LILRB2, LILRB4 or TIGIT, and the degree of inhibition was related to the concentration of the antibody, the higher the concentration, the more significant the degree of inhibition.

### Example 11: Sequencing of monoclonal antibody V-regions

Total RNA was extracted from hybridoma cells secreting anti-human SEMG1 using Trizol^{™} reagent (Invitrogen), and the heavy chain variable region (VH) gene and light chain variable region (VL) gene were amplified by reverse transcription polymerase chain reaction (RT-PCR) with family-specific primers. RT-PCR products were separated by agarose gel electrophoresis, and DNA of predicted size was gel purified and sequenced in forward and reverse directions. The PCR products were cloned into pGEM-T vector, and then transformed into JM109 bacteria. Individual colonies were picked for sequencing using universal primers. After the analysis of the sequencing results, further, the obtained DNA encoding the heavy chain variable region of the selected lead antibody was cloned into the in-frame pcDNA3.4-mIgG1 with the murine IgG1 constant region, and the DNA encoding light chain variable region was cloned into the in-frame plasmid pcDNA3.4-mκc with the murine κ constant region for light chain expression. After the extracted plasmid was transfected into 293E cells to express and to verify the activity, the amino acid sequences of VH and VL of the determined monoclonal antibody are shown in Table 4.

**Table 4 VH and VL sequences of monoclonal antibodies**

| Monoclonal Antibodies | VH | VL |
|---|---|---|
| 33-B5-C1-F5 | EVQLQESGAELVKPRASVK MSCKASGYTFSSYNMHWIR QTPEQGLEWIGAIYPGTGDT SYNQKFKGKATLTADKSSST AYMQLSSLTSEDSAVYYCAS AMDYWGQGTSVTVSS (Seq ID NO: 47) | DVVLTQTPLSLPVSLGDQASISCR SSQSLLHSNGNTYLQWFLQRSGQ SPKLLIYKVSNRFYGVPDRFSGSG SGTDFTLKISRVEAEDLGVYFCSQ SIDVPRTFGGGTKLEIK (Seq ID NO: 48) |
| 25-E10 -F8-B5 | QVQLQQPGAELVKPGASVK MSCKASGYTFTSYNMHWV KQTPGQGLEWIGAIYPGNG DTSYNQKFKGKATLTADKSS STAYMQLSSLTSEDSAVYYC ASAMDYWGQGTSVTVSS (Seq ID NO: 49) | SIVMTQTPLSLPVSLGDQASISCR SSQSLVHSNGNTYLQWYLQKPG QSPKLLIYKVSNRFSGVPDRFSGS GSGTDFTLKISRVEAEDLGVYFCS QSKHVPRTFGGGTKLEIK (Seq ID NO: 50) |
| 4-D9-G 6-H6 | QIQLVQSGPELKKPGETVKI SCKASGYTFTNYGMNWVK QSPGKGLKWMGGINTNTGE PTYNEEFKGRFAFSLETSAR TAYLRINNLKNEDTATYFCT RSVFALDYWGQGTSVTVSS (Seq ID NO: 51) | DVVMTQTPLSLPVSLGDQASISCR SSQSLVHSNGNTYLQWYLQKPG QSPKLLIYKVSNRFSGVPDRFSGS GSGTDFTLKISRVEAEDLGVYFCS QSKHVPRTFGGGTKLEIK (Seq ID NO: 52) |
| 4-D9-G 6-H6-1 | QIQLVQSGPELKKPGETVKI SCKASGYTFTNYGMNWVK QSPGKGLKWMGGINTNTGE PTYNEEFKGRFAFSLETSAR TAYLRINNLKNEDTATYFCT RSVFALDYWGQGTSVTVSS (Seq ID NO: 53) | DVVMTHTPLSLPVSLGDQASISCR SSQIILHSSGNTYLEWYLQKPGQS PKLLIYKVSNRFSGVPDRFSGSGS GTDFTLKISRVEAEDLGIYYCFQG SHVPGTFGGGTKLEIK (Seq ID NO: 54) |

The CDR region analysis of the VH and VL sequences of the monoclonal antibodies was performed online on the abysis website, and the amino acid sequences of the defined CDR regions are shown in Table 5.

**Table 5 CDR amino acid sequences of monoclonal antibodies**

| Antibodies | Chain | CDRs | | |
|---|---|---|---|---|
| | | CDR1 | CDR2 | CDR3 |
| 33-B5-C1-F5 | H | GYTFSSYN (Seq ID NO: 55) | IYPGTGDT (Seq ID NO: 56) | ASAMDY (Seq ID NO: 57) |
| | L | QSLLHSNGNT Y (Seq ID NO: 58) | KV (Seq ID NO: 59) | SQSIDVPRT (Seq ID NO: 60) |
| 25-E10-F8-B5 | H | GYTFTSYN (Seq ID NO: 61) | IYPGNGDT (Seq ID NO: (2) | ASAMDY (Seq ID NO: 57) |
| | L | QSLVHSNGNT Y (Seq ID NO: 63) | KV (Seq ID NO: 59) | SQSKHVPRT (Seq ID NO: 64) |
| 4-D9-G6-H6 | H | GYTFTNYG (Seq ID NO: 65) | NTNTGE (Seq ID NO: 66) | TRSVFALDY (Seq ID NO: 67) |
| | L | QSLVHSNGNT Y (Seq ID NO: 63) | KV (Seq ID NO: 59) | SQSKHVPRT (Seq ID NO: 64) |
| 4-D9-G6-H6-1 | H | GYTFTNYG (Seq ID NO: 65) | NTNTGE (Seq ID NO: 66) | TRSVFALDY (Seq ID NO: 67) |
| | L | QIILHSSGNTY (Seq ID NO: 68) | KV (Seq ID NO: 59) | FQGSHVPGT (Seq ID NO: 69) |

### Example 12: In vitro inhibitory effect of SEMG1 monoclonal antibody on tumor cells

In order to identify the inhibitory effect of the SEMG1 antibodies on tumor cells expressing SEMG1 or SEMG2, the following *in vitro* experiments were carried out: HCT116-SEMG1 or HCT116-SEMG2 tumor cells were selected, incubated with antibodies or control mouse IgG, and then co-incubated with PBMCs pre-activated by CD3 and CD28 for 12 hours, and the apoptosis ratio of CD45-tumor cells was analyzed by Annexin-V-labeled flow cytometry. The results showed that overexpression of SEMG1 or SEMG2 inhibited the killing of HCT116 tumor cells by activated PBMCs. SEMG1 monoclonal antibody acts on tumor cells overexpressing SEMG1 or SEMG2 to promote the killing effect of PBMC. The SEMG2-specific antibody MM02 isolated by our company before has a weaker effect on promoting PBMC killing than the antibody in the invention. Negative control monoclonal antibody did not promote killing. The results are shown in Figure 10. This shows that SEMG1 antibody plays a role by specifically inhibiting the function of SEMG1 or SEMG2; the hybridoma monoclonal antibody binding to SEMG1 and SEMG2 has a stronger killing effect on PBMC than the previously reported hybridoma monoclonal antibody of SEMG2; the expression of SEMG1 or SEMG2 is of great significance on the effect of killing of tumor cells by antibodies.

### Example 13: In vivo inhibitory effect of SEMG1 monoclonal antibody on tumor cells

In order to identify the inhibitory effect of the SEMG1 antibody on tumor cells expressing SEMG1 and SEMG2, a tumor-forming mouse *in vivo* experiment was carried out. Twelve 6-8 weeks old male NPSG mouse models were weighed. Human breast cancer cell MCF7 expressing SEMG1 and SEMG2 was cultured *in vitro* to obtain 1.8×10⁸ cells. After the mice were inoculated with PBMCs, they were inoculated with tumor cells on the third day, and then the proportion of hCD45+ cells in blood and body weight of mice were measured once a week. After inoculation, the tumor volume was measured once a week, and the proportion of hCD45+ cells in the mouse blood was measured when the average tumor volume reached about 40-80 mm³. The mice were randomly divided into groups according to the tumor volume and the proportion of hCD45+ cells in the blood of the mouse, and then the administration began. The start date of administration was deemed as Day 0. Administration regimen: intraperitoneal injection of SEMG1 antibody at 5 mg/kg three times a week. After the administration started, the tumor growth status of the mice was observed weekly. After the tumor growing, the body weight and tumor volume were measured 3 times a week, and the relative count of hCD45+ cells in the blood of the mice was monitored 3 times a week by flow cytometry. When the tumor volume reached the endpoint standard, blood was taken to detect the same indicators as above, and the experiment was ended. The observation of mice includes: daily observation, after inoculation, observation of animal morbidity and death every working day. Tumor volume measurement: after inoculation to before grouping, when the tumor is visible, the tumor volume of the experimental animals was measured once a week; after inoculation and grouping, the tumor volume of the animals in the experiment was measured twice a week. Tumor volume measurement adopts a two-way measurement method, the long and short diameter of the tumor were first measured with a vernier caliper, and then the tumor volume is calculated using the formula TV=0.5*a*b². In the formula, a is the long diameter of the tumor and b is the short diameter of the tumor. The results showed that: for MCF7 human breast cancer cells expressing SEMG1 and SEMG2, compared with the no antibody group, the SEMG1 antibody screened in the invention had a significant inhibitory effect on tumor growth in mice, and it was better than the inhibitory effect on tumor of the specific antibody MM02 against SEMG2 (FIG. 11). This shows that the SEMG1 antibody can be used to inhibit the inoculated SEMG1 and/or SEMG2-expressing tumors in experimental animals, and exhibits a good inhibitory effect. This indicates that tumor cells expressing SEMG1 and/or SEMG2 have a more significant response to SEMG1 antibodies, and the expression of specific target proteins SEMG1 or SEMG2 is of great significance for the targeted administration of SEMG1 antibodies.

The foregoing descriptions of specific exemplary embodiments of the present invention have been presented for purposes of illustration and description. These descriptions are not intended to limit the invention to the exact form as disclosed, and obviously many modifications and variations are possible in light of the above teaching. The exemplary embodiments were chosen and described in order to explain the specific principles of the invention and its practical application, thereby enabling the skilled person in the art to implement and utilize various exemplary embodiments and various aspects of the invention as well as various choices and changes. It is intended that the scope of the invention be defined by the claims and their equivalents.

## Claims

1. A specific antibody that binds to semenogelin, **characterized in that** the antibody is capable of binding to SEMG1 and SEMG2 proteins with high affinity, and regulate the binding of SEMG1 or SEMG2 to any receptor (LILRB2, LILRB4, TIGIT or CD27) thereof; the high affinity is defined as that the half effective concentration (EC50) value of antibody binding to SEMG1 obtained by ELISA detection is lower than 3.7 nanomolar, and the EC50 value of binding to SEMG2 is lower than 2.4 nanomolar, or the affinity is higher than affinity levels corresponding to EC50 values detected by other methods.

2. The specific antibody that binds to semenogelin of claim 1, wherein the preferred epitope of the antibody comprises the following amino acids from SEMG1 or SEMG2:
KDIFSTQDELLVYNK (amino acids 251-265 of SEMG1, SEQ ID NO: 26);
KDIFITQDELLVYNK (amino acids 251-265 of SEMG2, SEQ ID NO:70);
GHFHRWIHHKGGKA (amino acids 121-135 of SEMG1, SEQ ID NO: 13);
KGGKAHRGTQNPSQD (amino acids 131-145 of SEMG1, SEQ ID NO: 14),
AQKGRKQGGSQSSYV (amino acids 181- 195 of SEMG1, SEQ ID NO: 19), or
PNPKQEPWHGENAKG (amino acids 391-405 of SEMG1, SEQ ID NO: 40).

3. A class of antibodies competing for binding to SEMG1 or SEMG2 with antibodies having hypervariable region sequences (SEQ ID NOs: 47-54).

4. A method for identifying the antibody of claim 1 or 2, **characterized in that** at least one of the following potencies of the candidate antibody is analyzed: the strength of the antibody binding to isolated SEMG1 and SEMG2 proteins; the strength of the antibody binding to SEMG1 and SEMG2 proteins expressed on the cell surface; the effect of the antibody on SEMG1 binding to its receptor CD27; the effect of the antibody on SEMG1 binding to its receptor LILRB2; the effect of the antibody on SEMG1 binding to its receptor TIGIT; the effect of the antibody on SEMG1 binding to its receptor LILRB4; the effect of the antibody on SEMG2 binding to its receptor LILRB2; the effect of the antibody on SEMG2 binding to its receptor TIGIT; the effect of the antibody on SEMG2 binding to its receptor LILRB4; the effect of the antibody on myeloid-derived suppressor cells (MDSC); the effect of the antibody on regulatory T cells (Treg); the effect of the antibody on immune cells killing tumor cells.

5. A biomarker for determining whether to administer the antibody of claims 1-3, consisting of a combination of SEMG1 and SEMG2 expressions, the specific steps of use are: detecting the expressions of mRNA or protein of SEMG1 or SEMG2 in tumor cells or individual blood samples; when the expression of SEMG1 or SEMG2 is higher than that of normal cells or individuals, using the antibody of claims 1-3 to contact tumor cells *in vitro* or *in vivo,* detecting the expression of SEMG1 or SEMG2 after using the antibody for evaluating the inhibitory effect of the antibody.

6. A method for inhibiting tumor cells *in vitro* or *in vivo,* **characterized in** comprising the following steps: using the biomarker of claim 5 to analyze; using the antibody of claims 1-3 to contact SEMG1 or SEMG2 expressed by the tumor cells under appropriate conditions.

7. A method for inhibiting tumor cells *in vitro* and *in vivo,* **characterized in that** the function or activity of SEMG1 or SEMG2 is inhibited, including but not limited to the following methods: editing SEMG1 or SEMG2 genes; interfering SEMG1 or SEMG2 gene expression; inhibiting function or activity of SEMG1 or SEMG2 protein by antibodies.

8. A method for regulating the state of immune cells *in vitro* or *in vivo,* **characterized in that** the binding of SEMG1 or SEMG2 to receptors CD27, LILRB2, LILRB4 or TIGIT is promoted or inhibited.

9. A method for regulating the ratio of MDSC or Treg cells *in vitro* or *in vivo,* **characterized in that** the binding of SEMG1 or SEMG2 to receptors CD27, LILRB2, LILRB4 or TIGIT is promoted or inhibited.

10. A method for regulating the active state of CD27, LILRB2, LILRB4 or TIGIT receptors in cells *in vitro* or *in vivo,* **characterized in** promoting or inhibiting the binding of SEMG1 or SEMG2 to receptors CD27, LILRB2, LILRB4 or TIGIT.

11. A method for screening drugs or reagents for preventing or treating tumors, **characterized in that** the candidate drugs or reagents are obtained by screening inhibitors or antibodies that inhibit the interaction of SEMG1 or SEMG2 with receptors CD27, LILRB2, LILRB4 or TIGIT.

12. An agonistic or antagonistic compound, **characterized in that** the compound can regulate the interaction of SEMG2 with receptor CD27, LILRB2, LILRB4 or TIGIT; preferably, the compound is a small molecule inhibitor, polypeptide, antibody or antigen-binding fragment.

13. Any of the following uses of the antibody of claims 1-3:
use in antagonizing the interaction of SEMG1 or SEMG2 with CD27, LILRB2, LILRB4 or TIGIT; preferably, SEMG1 or SEMG2 is expressed in tumor cells, and CD27, LILRB2, LILRB4 or TIGIT is expressed in immune cells;
use in the preparation of a product for antagonizing the interaction of SEMG1 or SEMG2 with CD27, LILRB2, LILRB4 or TIGIT; preferably, SEMG1 or SEMG2 is expressed in tumor cells, and CD27, LILRB2, LILRB4 or TIGIT is expressed in immune cells;
use in preventing or treating tumors;
use in the preparation of a medicament for the prevention or treatment of tumors;
use in modulating the immune response elicited against tumors;
use in the preparation of a medicament for regulating immune responses against tumors;
use in inhibiting tumor cell growth *in vivo* and *in vitro;*
use in the preparation of a reagent for inhibiting tumor cell growth *in vivo* and *in vitro;*
preferably, the inhibitor can inhibit or block the binding of SEMG1 or SEMG2 to its receptor CD27, LILRB2, LILRB4 or TIGIT;
more preferably, the inhibitor is a small molecule inhibitor, polypeptide, antibody or antigen-binding fragment.

14. A method for preventing or treating tumors, comprising:
contacting immune cells or tumor cells of a subject with an effective dosage of a SEMG1 or SEMG2 antibody; the antibody is capable of inhibiting the binding of SEMG1 or
SEMG2 to its receptor CD27, LILRB2, LILRB4 or TIGIT;
preferably, the subject has received or is receiving or will receive additional anticancer therapy;
more preferably, the additional anticancer therapy comprises surgery, radiotherapy, chemotherapy, immunotherapy or hormone therapy.

15. A companion diagnostic method for tumor immunotherapy, comprising: detecting the SEMG1 or SEMG2 protein expression amount or function of tumor cells of a subject before/after immunotherapy.

16. An isolated antibody or antigen-binding fragment thereof, **characterized in that**:
a) the antibody or antigen-binding fragment thereof specifically binds SEMG1 or SEMG2 protein with high affinity; and
b) the antibody is capable of inhibiting or blocking the interaction of SEMG1 or SEMG2 with any of CD27, LILRB2, LILRB4 or TIGIT;
preferably, the antibody further comprises at least one of the following properties:
i. the antibody reduces the ratio of immunosuppressive cells MDSC cells;
ii. the antibody reduces the cell ratio of immunosuppressive cells Treg; or
iii. the antibody enhances the killing effect of immune cells (such as PBMC) on tumor cells.

17. An isolated antibody or antigen-binding fragment thereof, **characterized in** comprising three CDRs in the amino acid sequence of the heavy chain variable region as shown in SEQ ID NOs: 47, 49, 51, 53 and three CDRs in the amino acid sequence of the light chain variable region as shown in SEQ ID NOs: 48, 50, 52 and 54; or a variant with no more than two amino acid variations in each CDR region in single or multiple CDRs as compared with the above six CDR regions.

18. The antibody or antigen-binding fragment of claim 17, **characterized in** comprising a heavy chain variable region comprising HCDR1, HCDR2 and HCDR3 sequences defined according to general method; and a light chain variable region comprising LCDR1, LCDR2 and LCDR3 sequences defined according to general method, the antibody or antigen-binding fragment comprises the following CDR sequences:
the amino acid sequence of the HCDR1 is shown in any one of SEQ ID NOs: 55, 61, 65;
the amino acid sequence of the HCDR2 is shown in any one of SEQ ID NOs: 56, 62, 66;
the amino acid sequence of the HCDR3 is shown in any one of SEQ ID NOs: 57, 67;
the amino acid sequence of the LCDR1 is shown in any one of SEQ ID NOs: 58, 63, 68;
the amino acid sequence of the LCDR2 is shown in SEQ ID NO: 59;
the amino acid sequence of the LCDR3 is shown in any one of SEQ ID NOs: 60, 64, 69;
or a variant with no more than two amino acid variations in each CDR region in single or multiple CDRs as compared with the six CDR regions of above;
preferably, the binding K_{D} of the antibody or its antigen-binding fragment to SEMG1 or SEMG2 is <2×10⁻⁸; more preferably, the K_{D}<2×10⁻⁸, <1×10⁻⁸, <9×10⁻⁹, <8×10⁻⁹, <7×10⁻⁹, <6×10⁻⁹, < 5×10⁻⁹, <4×10⁻⁹, <3×10⁻⁹, <2×10⁻⁹, <1×10⁻⁹, <1×10⁻¹⁰.

19. The antibody or antigen-binding fragment of claims 17-18, **characterized in that** the antibody or antigen-binding fragment comprises a heavy chain variable region and a light chain variable region, and the sequences are selected from the following:
a) the amino acid sequence of the heavy chain variable region comprises an amino acid sequence selected from the group consisting of SEQ ID NOs: 47, 49, 51, 53 or has at least 70%, 80%, 90%, 95% or 99% sequence identity to the sequence in the group;
b) the amino acid sequence of the light chain variable region comprises an amino acid sequence selected from the group consisting of SEQ ID NOs: 48, 50, 52, 54 or has at least 70%, 80%, 90%, 95% or 99% sequence identity to the sequence in the group.

20. The antibody or antigen-binding fragment of claims 16-19, **characterized in that** the antibody further comprises a conjugation moiety connected to a polypeptide, the conjugation moiety is selected from one or more of radionuclides, drugs, toxins, cytokines, enzymes, fluoresceins, carrier proteins, lipids, and biotins, wherein the polypeptide or antibody and the conjugation moiety is optionally connected by a linker, preferably, the linker is a peptide or polypeptide;
more preferably, the antibody is selected from monoclonal antibody, polyclonal antibody, antiserum, chimeric antibody, humanized antibody and human antibody; more preferably, the antibody is selected from multispecific antibody, single chain Fv (scFv), single chain antibody, anti-idiotypic (anti-Id) antibody, diabody, minibody, nanobody, single domain antibody, Fab fragment, F(ab') fragment, disulfide-linked bispecific Fv (sdFv) and intrabody.

21. An isolated polynucleotide, **characterized in that** the polynucleotide encodes the antibody or antigen-binding fragment of any one of claims 17-20.

22. A recombinant vector comprising the polynucleotide of claim 21, and optional regulatory sequence;
preferably, the recombinant vector is a cloning vector or an expression vector;
more preferably, the regulatory sequence is selected from a leader sequence, a polyadenylation sequence, a propeptide sequence, a promoter, a signal sequence, a transcription terminator, or any combination thereof.

23. A host cell, **characterized in** comprising the recombinant vector of claim 22;
preferably, the host cell is a prokaryotic cell or a eukaryotic cell.

24. A pharmaceutical composition, **characterized in** comprising one or more of the antibody or antigen-binding fragment, polynucleotide, recombinant vector and host cell of any one of claims 17-23;
preferably, the composition further comprises a pharmaceutically acceptable carrier or adjuvant.

25. A kit, **characterized in** comprising one or more of the antibody or antigen-binding fragment, polynucleotide, recombinant vector, host cell and pharmaceutical composition of any one of claims 17-24, and contained in a suitable container.

26. Any of the following uses of the antibody or antigen-binding fragment, polynucleotide, recombinant vector, host cell and pharmaceutical composition of any one of claims 17-24:
use in agonizing or antagonizing the interaction of SEMG1 or SEMG2 with its receptor CD27, LILRB2, LILRB4 or TIGIT; preferably, SEMG1 or SEMG2 is expressed in tumor cells, and CD27, LILRB2, LILRB4 or TIGIT is expressed in immune cells;
use in the preparation of a product for agonizing or antagonizing the interaction of SEMG1 or SEMG2 with its receptor CD27, LILRB2, LILRB4 or TIGIT; preferably,
SEMG1 or SEMG2 is expressed in tumor cells, and CD27, LILRB2, LILRB4 or TIGIT is expressed in immune cells;
use in preventing or treating tumors;
use in the preparation of a medicament for the prevention or treatment of tumors;
use in modulating the immune response elicited against tumors;
use in the preparation of a medicament for regulating immune responses against tumors;
use for inhibiting tumor cell growth *in vivo* and *in vitro*;
use in the preparation of a reagent for inhibiting tumor cell growth *in vivo* and *in vitro.*

27. A method for preventing or treating tumors, comprising:
contacting immune cells and tumor cells of a subject with an effective dosage of the antibody or antigen-binding fragment, polynucleotide, recombinant vector, host cell and pharmaceutical composition of any one of claims 17-24;
preferably, the expression of SEMG1 or SEMG2 in the tumor cells is detected before the effective amount of the compound is used to contact the immune cells and/or tumor cells of the subject;
preferably, the immune cells are lymphocytes, more preferably, T lymphocytes;
further preferably, the subject has received or is receiving or will receive additional anticancer therapy;
even more preferably, the additional anticancer therapy comprises surgery, radiotherapy, chemotherapy, immunotherapy or hormone therapy.

28. A method for detecting the presence or absence of SEMG1 or SEMG2 in a biological sample *in vivo* and *in vitro,* **characterized in** contacting the biological sample with the antibody or antigen-binding fragment, polynucleotide, recombinant vector, host cell and pharmaceutical compound of any one of claims 17-24.

29. A method for inhibiting tumor cell growth, comprising the steps of:
A) analyzing the expression of SEMG1 or SEMG2 in tumor cells;
B) using an antibody that can recognize SEMG1 or SEMG2 to contact with tumor cells, and the binding K_{D} of the antibody to SEMG1 or SEMG2 is <2×10⁻⁸; preferably, the K_{D}<2×10⁻⁸, <1×10⁻⁸, <9×10⁻⁹, <8×10⁻⁹, <7×10⁻⁹, <6×10⁻⁹, <5×10⁻⁹, <4×10⁻⁹, <3×10⁻⁹, <2×10⁻⁹, <1×10⁻⁹, <1×10⁻¹⁰;
C) contacting T lymphocytes, the antibody with tumor cells.

30. Any one of the preceding claims, **characterized in that** the tumor is selected from colorectal cancer, lung cancer, breast cancer, melanoma, lymphoma, liver cancer, head and neck cancer, gastric cancer, kidney cancer, bladder cancer, prostate cancer, testicular cancer, endometrial cancer, breast cancer and ovarian cancer.
